# EUROPEAN PATENT APPLICATION

(11) **EP 2 754 451 A1**
(43) Date of publication of application: **16.07.2014**
(21) Application number: 12821830.2
(22) Date of filing: 08.08.2012
(51) Int. Cl.: A61K 38/46, C12N 15/55, C12N 15/63, C12N 9/16, C12Q 1/46, A61P 35/00, A61P 43/00

(54) **THE USE OF ACHE AS NUCLEASE**

(30) Priority: 08.08.2011 CN 201110226287
(71) Applicant: Shanghai Institutes for Biological Sciences, Chinese Academy of Sciences, Shanghai 200031 (CN)
(72) Inventor: ZHANG, Xuejun, Shanghai 200031 (CN); XIE, Jing, Shanghai 200031 (CN); DU, Aiying, Shanghai 200031 (CN); GUO, Kaijie, Shanghai 200031 (CN); WU, Jun, Shanghai 200031 (CN)
(74) Representative: Dupuis-Latour, Dominique
(86) International application number: PCT/CN2012/001056
(87) International publication number: WO 2013/020366

(57) **Abstract**

This invention provides the use of AChE as nuclease. The use of AChE in regulating the stability of nucleic acid and cell apoptosis, as well as a series of agents based on the use, including the agents for promoting or inhibiting cell apoptosis, the agents for inhibiting tumors, and the agents for protecting nucleic acid from impairing, are provided.

## Description

### TECHNICAL FIELD

The present invention relates to the field of biotechnology, more particularly, to the use of acetylcholinesterase as nuclease.

### BACKGROUND ART

Cell apoptosis is a very important physiological process for organisms, especially for higher organism. Due to the presence of cell apoptosis, the formation of organs and morphological structures during development process and dynamic maintain of reasonable cell number as well as clearance of undesired or even harmful cells (such as tumor) become possible.

Significant signs during cell apoptosis are chromosome breakage and nucleic acid degradation. Generally, in apoptotic cells, after chromosome condensation, chromosome is first degraded into large DNA fragments of 50 - 300 kb in length, and then broken at the junction of nucleosomes regularly, forming DNA fragments of 180 - 200 bp or integral multiples of 200 bp, before phagocytosis of apoptotic bodies containing fragmented DNA by macrophages or neighbouring cells, and the complete degradation of DNA in the end, which makes the cell completely lose the abilities of genome replication and gene transcription, resulting in irreversible apoptosis. It is indicated through biochemical and genetic researches that chromosome DNA degradation during cell apoptosis process is a very complicated biochemical process involving various nucleases with different activities and different substrate specificities. During apoptosis process, interactions between various nucleases described above or between the nucleases and other cofactors promote chromosome breakage and DNA degradation progressively. Although this process is crucial, how is it activated, and how is it specifically carried out? Whether the degradation of chromosomal DNA is the reason or the result of apoptosis? These questions have not been fully explained so far. In addition, although *in vivo* experiments have already confirmed that a part of nucleases are involved in this process, enzymes correlative to the degradation of chromosomal DNA during apoptosis process are far more than what we have found, and there are still many other enzymes participating in the degradation of chromosomal DNA under specific conditions within an apoptotic cell.

Currently known DNases involved in the degradation of chromosomal DNA during apoptosis in mammalian cells are mainly CAD/DFF40 (Caspase activated deoxyribonuclease/ 40-kDa DNA fragmentation factor), AIF (apoptosis inducing factor) and Endo G (Endonuclease G) 4. These three enzymes are significantly different in spatial distribution and substrate preference. In cells growing normally, CAD/DFF40 is mainly distributed in cytoplasm, which forms a complex with its inhibitor ICAD/DFF45 and is kept in a silence state. When apoptosis is started, ICAD/DFF45 is inactivated by cleavage under the effect of activated Caspase 3/7, releasing free CAD. After entering cell nucleus, CAD promotes the DNA cleavage between nucleosomes with the help of chromosomins such as Histone H1, high mobility group protein (HMG) and Topoisomerase II, forming DNA fragments containing hydroxy at the 3' end (3'-OH). AIF was first considered as an oxidordeuctase in mitochondria. When cell is stimulated by apoptosis, due to the change in membrane permeability of mitochondria, AIF is released outside of the mitochondria and enters cell nucleus, causing chromosome condensation and production of large broken fragments of DNA (50 kDa). Since AIF is provided with no classic nuclease domain nor documented nuclease activity, it is thought to function in chromosome fragmentation through a certain nuclease effector. Similar to AIF, Endo G is also a nuclease found in mitochondria independent of Caspase activation. When a cell is stimulated by apoptosis, Endo G is released outside of mitochondria and enters cell nucleus, leading to the breakage of condensed chromosomal DNA. In DFF45 deficient mice, the residual DNA fragmentation is mediated by Endo G. It is generally considered that Endo G functions as a facilitator of CAD/DFF40 during the DNA clearance in apoptotic cells, and furthermore, it also participates in the subsequent clearance of nucleosomal DNA. *In vitro* experiments have found that, compared with CAD/DFF40, Endo G needs a higher concentration when inducing DNA degradation (100 times higher than CAD/ DFF40). It is assumed that the effect of Endo G alone is not enough, and participation of further nucleases or cofactors is needed; the two mitochondrial proteins, Endo G and AIF, may be needed in the limited activation or injury of Caspase in order to ensure the degradation of the nucleus. In a word, nucleus can act synergistically *in vivo* to ensure effective DNA degradation in apoptotic cells. Although gene knockout directed to the three enzymes can effectively inhibit the process of apoptotic DNA clearance, it cannot completely prevent the process from occurring. For example, in CAD/DFF40 deficient mammalian cells, we can still observe the breakage and fragmentation of chromosomal DNA, indicating that this process involves other nucleases.

The inventor has found that increased expression of acetylcholinesterase (AChE) can always be observed when using different methods to induce various cells to apoptosis. However, the function of AChE in cell apoptosis is still not clear yet. Thus, it is necessary to further investigate the role of AChE in cell apoptosis, in order to develop new ways to regulate cell events (such as cell apoptosis) based on this.

### SUMMARY OF THE INVENTION

The present invention aims at providing the use of acetylcholinesterase as nuclease.

In the first aspect of the present invention, provided is the use of acetylcholinesterase or agonist or antagonist thereof in preparation of a composition (such as a drug) for regulating the stability of nucleic acid (e.g., DNA) or regulating cell apoptosis.

In a preferred embodiment, said acetylcholinesterase is used as nuclease.

In another preferred embodiment, said acetylcholinesterase is: S form of acetylcholinesterase (AChES), R form of acetylcholinesterase (AChER) or H form of acetylcholinesterase (AChEH).

In another preferred embodiment, said acetylcholinesterase is selected from the group consisting of:
(a) a polypeptide corresponding to SEQ ID NO: 5;
(b) a polypeptide corresponding to aa 32-138 of SEQ ID NO:5;
(c) a polypeptide corresponding to aa 2-138 of SEQ ID NO:5;
(d) a polypeptide corresponding to aa 2-191 of SEQ ID NO:5;
(e) a polypeptide corresponding to aa 2-247 of SEQ ID NO:5;
(f) a polypeptide corresponding to aa 2-398 of SEQ ID NO:5;
(g) a polypeptide corresponding to aa 32-578 of SEQ ID NO:5;
(h) a polypeptide corresponding to aa 32-579 of the amino acid sequence represented by GenBank accession number NP_033729.1;
(i) a polypeptide with amino acid sequence from any of polypeptides (a), (e)-(g), with variations occurring in position 234, 365 and/or 478 (such as S234A, E365A, H478A);
(j) a polypeptide formed through substitution, deletion or addition of one or more (e.g. 1 - 20, preferably 1 - 15, more preferably 1 - 10, more preferably 1 - 5, even more preferably 1 - 3) amino acid residues in the amino acid sequences from any of (a)-(i) (i.e. any one selected from (a), (b), (c), (d), (e), (f), (g), (h) or (i)), derived from (a) and possessing the function of polypeptide (a);
(k) fragment of polypeptide from any of (a)- (j) (i.e. any one selected from (a), (b), (c), (d), (e), (f), (g), (h), (i) or (j)), possessing the function of polypeptide (a), (preferably, having greater than 20% sequence identity to SEQ ID NO: 5; more preferably greater than 30%; more preferably greater than 40%; more preferably greater than 50%; more preferably greater than 60%; more preferably greater than 70%; more preferably greater than 80%; more preferably greater than 85%; more preferably greater than 90%; more preferably greater than 95%; even more preferably greater than 98% or 99% identity to SEQ ID NO: 5); or
(l) a polypeptide possessing the function of polypeptide (a), containing the amino acid sequence of any of polypeptides (a)-(k).

In another preferred embodiment, a polypeptide only containing aa 2-72 of SEQ ID NO: 5, or the truncated form thereof is not included in (i) or (k).In another preferred embodiment, said acetylcholinesterase or the agonist thereof is used in preparation of composition for damaging nucleic acid and/or promoting cell apoptosis.

In another preferred embodiment, said acetylcholinesterase agonist is selected from the group consisting of: agents which direct acetylcholinesterase or its encoding gene to enter the nucleus (such as nuclear importer), magnesium ion, calcium ion or materials which can form magnesium ion and/or calcium ion.

In another preferred embodiment, said acetylcholinesterase agonist is the agonist promoting acetylcholinesterase to damage nucleic acid or promoting acetylcholinesterase to induce cell apoptosis.

In another preferred embodiment, said nucleic acid is tumor- or virus-related nucleic acid (e.g., tumor cell genome DNA, viral genome DNA or RNA), or said cell is a tumor cell.

In another preferred embodiment, said acetylcholinesterase or the agonist thereof is used in preparation of composition for tumor inhibition.

In another preferred embodiment, said acetylcholinesterase inhibitor is used in preparation of composition for nucleic acid protection and/or cell apoptosis inhibition.

In another preferred embodiment, said inhibitor is selected from the group consisting of: nucleic acid inhibitors, protein inhibitors, antibodies, ligands, proteolytic enzymes, protein-binding molecules, EDTA.

In another preferred embodiment, said nucleic acid inhibitor is selected from the group consisting of: interfering molecule according to SEQ ID NO: 1 or SEQ ID NO: 2.

In another preferred embodiment, said acetylcholinesterase inhibitor is the inhibitor which represses the regulation of nucleic acid stability or the regulation of cell apoptosis-related activity by acetylcholinesterase.

In another preferred embodiment, the molecular weight of said acetylcholinesterase is preferably less than 80kD; more preferably less than 70KD; more preferably less than 60KD.

In another aspect of the present invention, provided is an isolated acetylcholinesterase fragment or variant, which is selected from the group consisting of:
(b) a polypeptide corresponding to aa 32-138 of SEQ ID NO:5;
(c) a polypeptide corresponding to aa 2-138 of SEQ ID NO:5;
(d) a polypeptide corresponding to aa 2-191 of SEQ ID NO:5;
(e) a polypeptide corresponding to aa 2-247 of SEQ ID NO:5;
(f) a polypeptide corresponding to aa 2-398 of SEQ ID NO:5;
(g) a polypeptide corresponding to aa 32-578 of SEQ ID NO:5;
(h) a polypeptide corresponding to aa 32-579 of the amino acid sequence represented by GenBank accession number NP_033729.1;
(i) a polypeptide with amino acid sequence from any of polypeptides (e)-(g), with variations occurring in position 234, 365 and/or 478 (such as S234A, E365A, H478A);
(j) a polypeptide formed through substitution, deletion or addition of one or more (e.g. 1 - 20, preferably 1 - 15, more preferably 1 - 10, more preferably 1 - 5, more preferably 1 - 3) amino acid residues in any one amino acid sequence from polypeptides (b)-(i) (i.e. any one selected from (b), (c), (d), (e), (f), (g), (h) and (i)), possessing the function of the polypeptide corresponding to SEQ ID NO: 5;
(k) fragments from any of polypeptides (b)-(j) (i.e. any one selected from (b), (c), (d), (e), (f), (g), (h), (i) or (j)), possessing the function of the polypeptide corresponding to SEQ ID NO: 5 (preferably, having greater than 20% sequence identity to SEQ ID NO: 5; more preferably greater than 30%; more preferably greater than 40%; more preferably greater than 50%; more preferably greater than 60%; more preferably greater than 70%; more preferably greater than 80%; more preferably greater than 85%; more preferably greater than 90%; more preferably greater than 95%; more preferably greater than 98% or 99% sequence identity to SEQ ID NO: 5); or
(l) a polypeptide having the function of the polypeptide corresponding to SEQ ID NO: 5, containing amino acid sequence from any of polypeptides (b)-(k).

In another preferred embodiment, the polypeptide only containing aa 2 - 72 of SEQ ID NO: 5 or the truncated form thereof is not included in (i), (j) or (k).

In another preferred embodiment, said acetylcholinesterase fragments or variants do not include the acetylcholinesterase in full-length (such as the acetylcholinesterase of SEQ ID NO: 5).

In another aspect of the present invention, provided is an isolated polynucleotide containing one nucleotide sequence, wherein the nucleotide sequence is selected from the group consisting of:
(1) a polynucleotide encoding said polypeptide;
(2) a polynucleotide complementary to polynucleotide (1).

In another preferred embodiment, the nucleotide sequence of the polynucleotide is selected from (but not limited to):
(i) a polynucleotide having the nucleotide sequence from position 4 to 414 of SEQ ID NO:4; (the polypeptide corresponding to aa 2-138 of SEQ ID NO:5);
(ii) a polynucleotide having the nucleotide sequence from position 4 to 573 of SEQ ID NO:4; (the polypeptide corresponding to aa 2-191 of SEQ ID NO:5);
(iii) a polynucleotide having the nucleotide sequence from position 4 to 741 of SEQ ID NO:4; (the polypeptide corresponding to aa 2-247 of SEQ ID NO:5);
(iv) a polynucleotide having the nucleotide sequence from position 4 to 1194 of SEQ ID NO:4; (the polypeptide corresponding to aa 2-398 of SEQ ID NO:5); or
(v) a polynucleotide having the nucleotide sequence from position 94 to 1734 of SEQ ID NO:4; (the polypeptide corresponding to aa 32-578 of SEQ ID NO:5).

In another aspect of the present invention, provided is a vector which contains said polynucleotide.

In another aspect of the present invention, provided is a genetic engineered host cell which contains said vector; or the genome of the cell is integrated with said polynucleotide.

In another aspect of the present invention, provided is a method for preparing said acetylcholinesterase fragment or variant, wherein the method comprises:
(a) culturing said host cell under conditions suitable for expression;
(b) isolating said acetylcholinesterase fragment or variant from the culture.

In another aspect of the present invention, provided is the use of said acetylcholinesterase fragment or variant, said polynucleotide, said vector or said host cell, for preparing compositions to regulate nucleic acid (such as DNA) stability or cell apoptosis, or preparing anti-tumor drugs.

In another aspect of the present invention, provided is an acetylcholinesterase inhibitor for protecting nucleic acid and/or inhibiting cell apoptosis, which has the nucleotide sequence of SEQ ID NO: 1 or SEQ ID NO: 2.

In another aspect of the present invention, provided is a material to regulate nucleic acid stability or cell apoptosis, which comprises:
(1) acetylcholinesterase or the agonist or antagonist of acetylcholinesterase; and
(2) nuclear importer for directing acetylcholinesterase or the agonist or antagonist of acetylcholinesterase into the nucleus.

In another preferred embodiment, said acetylcholinesterase is: S form of acetylcholinesterase (AChES), R form of acetylcholinesterase (AChER) or H form of acetylcholinesterase (AChEH).

In another preferred embodiment, said material further comprises:
(3) cell- or tissue-targeting materials; and/or
(4) a protein transduction domain polypeptide.

In another preferred embodiment, said cell-targeting material is selected from (but not limited to): binding molecules which recognize surface molecules of specific cells (e.g. antibody (preferably monoclonal antibody), ligand, small chemical molecule or polypeptide).

In another preferred embodiment, the protein transduction domain polypeptide is selected from (but not limited to): TAT, Antp, VP22 or Low Molecular Weight Protamine (LMWP).

In another preferred embodiment, said acetylcholinesterase is covalently or non-covalently linked to the protein transduction domain polypeptide.

In another preferred embodiment, among said materials, (1), (2), (3) and/or (4) combine with each other by means of covalent linkage, coupling, molecular force, charge adsorption, adherence, encapsulation or embedding.

In another preferred embodiment, said nuclear importer is selected from (but not limited to): a nuclear localization signal or karyopherin or core fragments thereof (retaining the function of nuclear localization or nuclear transport), steroid hormone, virus and viral protein or virus-like particle, cationic polymer, radionuclide, nanosphere.

Alternatively, the nuclear importer is a nuclear localization signal sequence or fragment of the nuclear localization signal sequence having the function of nuclear localization, the nuclear localization signal sequence includes: Simian Virus 40 (SV40) large T NLS, M9 NLS, c-myc NLS, nucleoplasmin NLS, Xenopus N1 NLS, FGF3 NLS, and PARP NLS, antennapedia peptide, TAT peptide, c-myc peptide, VirD2 peptide, nucleoplasmin peptide, aryl hydrocarbon receptor nuclear translocator (ARNT) peptide, a polyoma large T antigen nuclear localization signal sequence, an adenovirus E1a nuclear localization signal sequence, and an adenovirus E1b nuclear localization signal sequence, the adenoviral NLS, the HIV-I Tat protein NLS, the lymphoid enhancer factor 1 NLS, an IBD-oligolysine peptide, a dual function CD46 targeting peptide/adenoviral NLS, H2B, v-Jun, Dorsal, NIN2, SWI5, RB, PTHrP, HnRNP, Pho4, rpL23a, rpL25,Protamine NLS, MTA1, CBP80, Rev, STAR NLS, hTAP, Mata2 NL.

Alternatively, the virus, viral protein or virus-like particle are selected from (but not limited to): JC virus, JC virus VP1 protein, JC virus-like particle (VLP), chicken anemia virus, chicken anemia virus VP3, Apoptin.

Alternatively, the cationic polymer is selected from (but not limited to): β -carboxyl amidated cationic polymers such as polyethyleneimine (PEI) and Poly-L-Lysine (PLL).

Altrenatively, the nuclear importer includes (but not limited to): Auger-emitting radionuclides such as (99m)Tc, persulfated molecular umbrella, glucose-derived carbon nanospheres, estradiol-pheophorbide.

In another preferred embodiment, said nuclear localization signal sequence is, for example, SEQ ID NO: 3.

In another preferred embodiment, said material is a fusion protein which includes acetylcholinesterase and nuclear importer.

Alternatively, said fusion protein consists of acetylcholinesterase and nuclear importer.

Alternatively, there is a linker sequence (such as linker peptide) between the acetylcholinesterase and nuclear importer or not.

In another preferred embodiment, said fusion protein comprises: acetylcholinesterase (having core sequences) and nuclear localization signal (having core sequences).

In another preferred embodiment, said nuclear localization signal is selected from: aa 1-19 or aa 2-19 or aa 3-10 of SEQ ID NO:6.

In another preferred embodiment, said fusion protein has an amino acid sequence of SEQ ID NO:6.

In another preferred embodiment, said nuclear localization signal can be the nuclear localization signal of single repeating unit, or of multiple (e.g., 1-20; preferably 1-10; more preferably 1-5; more preferably 1-3) tandem repeating units.

In another aspect of the present invention, provided is a polynucleotide encoding any above-mentioned material (fusion protein).

In another aspect of the present invention, provided is an expression construct (such as plasmid vector, virus vector) which contains a polynucleotide encoding said material (fusion protein).

In another preferred embodiment, said expression construct further includes specific promoter operably linked to the polynucleotide of said fusion protein, wherein said specific promoter is selected from: cell (such as cancer cell)-specific promoter, tissue-specific promoter, or nucleus-specific promoter.

In another preferred embodiment, said promoter is a tumor cell-specific promoter, which is selected from (but not limited to): carcinoembryonic antigen (CEA) promoter, alpha fetoprotein (AFP) promoter, survivin(SUR), human telomerase reverse transcriptase (hTERT) gene promoter, secretory leukoprotease inhibitor (SLPI) promoter, squamous cell carcinoma antigen 2 (SCCA2) promoter, prostate cancer-specific promoter PSAe(AREc)-PSMAe-TARPp[P(A)PTp], prostate specific antigen (PSA), tyrosine kinase gene promoter, 5'-end specific promoter of tyrosinase protein 1 gene, gene promoter of epithelial mucin (MUC)-1, tumor drug resistance gene promoter, tissue-specific promoter OSP-1(ova-specific promoter-1), PSMA promoter, breast cancer-specific gene 1 (BCSG1) promoter, multi-drug resistance gene promoter.

In another aspect of the present invention, provided is the use of said material, polynucleotide encoding said material or expression construct comprising said polynucleotide, for (entering cell nucleus,) damaging nucleic acid and / or promoting cell apoptosis; or for producing composition to damage nucleic acid and / or promote cell apoptosis.

In another preferred embodiment, said nucleic acid is tumor- or virus-related nucleic acid, or said cell is a tumor cell.

In another preferred embodiment, said material is used for preparing antitumor drug.

In another aspect of the present invention, provided is a composition for damaging nucleic acid and / or promoting cell apoptosis, which comprises: said acetylcholinesterase fragment or variant, or said vector, or said material, or said polynucleotide encoding said material, or an expression construct comprising said polynucleotide; and
a pharmaceutically or physiologically acceptable vector.

In another preferred embodiment, said composition inhibits tumor or virus.

In another preferred embodiment, said composition for damaging nucleic acid and / or promoting cell apoptosis is an antitumor drug.

A composition for damaging nucleic acid and / or promoting cell apoptosis, which comprises: acetylcholinesterase, said acetylcholinesterase fragment or variant, or said vector, or said material, or said polynucleotide, or said expression construct; and
magnesium ions and/or calcium ions.

In another preferred embodiment, the pH value of said composition is 5-10.

In another preferred embodiment, the pH value of said composition is 7-10.

In another preferred embodiment, said composition is a pharmaceutical composition, the pH value of which is generally about 5-8, preferably, about 6-8, more preferably, about 7-8.

In another aspect of the present invention, provided is a method for damaging (such as cleaving) nucleic acid (preferably, a non-therapeutic method), which comprises: contacting the nucleic acid with acetylcholinesterase (including its fragment or variant) or the agonist thereof, or vectors expressing acetylcholinesterase or the agonist thereof, or said acetylcholinesterase fragment or variant, or said vector.

In another aspect of the present invention, provided is a method for promoting cell apoptosis (preferably, a non-therapeutic method), which comprises:
treating cells with acetylcholinesterase (including its fragment or variant) or the agonist thereof, or vectors expressing acetylcholinesterase (including its fragment or variant), or said material, or said polynucleotide encoding said material, or said expression construct comprising the polynucleotide; or
expressing acetylcholinesterase (including its fragment or variant) or the agonist thereof, or said material; or said acetylcholinesterase fragment or variant in the cells; or
directing acetylcholinesterase (including its fragment or variant) or said acetylcholinesterase fragment or variant into the nucleus.

In another aspect of the present invention, provided is a method for protecting nucleic acid or inhibiting cell apoptosis (preferably, a non-therapeutic method), which comprises:
treating cells with inhibitor of acetylcholinesterase (including its fragment or variant) or the agonist thereof, particularly, suppressive drugs designed for nuclease active site(e.g., position 2-138 of SEQ ID NO: 5);
expressing the inhibitor of acetylcholinesterase in the cells; or
preventing acetylcholinesterase from entering the nucleus.

In another aspect of the present invention, provided is a method for screening potential drugs which can regulate cell apoptosis or nucleic acid stability, wherein the method comprises:
(1) treating a system expressing or containing acetylcholinesterase or its fragment or variant with candidate materials; and
(2) detecting the transcription, expression or activity of the acetylcholinesterase or its fragment or variant in said system;

Wherein, if said candidate materials can improve (preferably significantly improve, such as improve by more than 20%, preferably improve by 50%; more preferably improve by greater than 80%) the transcription, expression or activity of acetylcholinesterase or its fragment or variant, it indicates that the candidate materials are potential materials to damage nucleic acid or promote cell apoptosis; if said candidate materials can reduce (preferably significantly reduce, such as reduce by more than 20%, preferably reduce by 50%; more preferably reduce by greater than 80%) the transcription, expression or activity of acetylcholinesterase or its fragment or variant, it indicates that the candidate materials are potential materials to protect nucleic acid or inhibit cell apoptosis.

In another preferred embodiment, said system expressing or containing acetylcholinesterase or its fragment or variant is a cell, and step (2) further includes:
observing nuclear transportation of acetylcholinesterase or its fragment or variant or its encoding gene; if the proportion of the acetylcholinesterase or its fragment or variant or its encoding gene in the nucleus increases (preferably significantly increases, such as increases by more than 20%, preferably increases by 50%; more preferably increases by greater than 80%), it suggests that the candidate materials are potential materials to damage nucleic acid or promote cell apoptosis; if the proportion of the acetylcholinesterase or its fragment or variant or its encoding gene in cell nucleus decreases (preferably significantly decreases, such as decreases by more than 20%, preferably decreases by 50%; more preferably decreases by greater than 80%), it suggests that the candidate materials are potential materials to protect nucleic acid or inhibit cell apoptosis; or,
step (2) further includes:
   observing DNA breakage in cell nucleus; if DNA breakage increases (preferably significantly increases, such as increases more than 20%, preferably increases 50%; more preferably increases greater than 80%), it suggests that the candidate materials are potential materials to damage nucleic acid or promote cell apoptosis; if DNA breakage decreases (preferably significantly decreases, such as decreases by more than 20%, preferably decreases by 50%; more preferably decreases by greater than 80%), it suggests that the candidate materials are potential materials to protect nucleic acid or inhibit cell apoptosis.
   In another preferred embodiment, step (1) includes: in a test group, adding candidate materials to the system expressing or containing acetylcholinesterase or its fragment or variant; and / or
step (2) includes: detecting the transcription, expression, activity or transportation to nucleus of the acetylcholinesterase or its fragment or variant in the system of the test group, and comparing with that of a control group, wherein said control group is a system expressing or containing the acetylcholinesterase or its fragment or variant without adding said candidate materials;

If the transcription, expression, activity or transportation to nucleus of the acetylcholinesterase or its fragment or variant in the test group get statistically improved (preferably significantly improved, such as get improved by more than 20%, preferably improved by 50%; more preferably improved by greater than 80%), it suggests that the candidate materials are potential materials to damage nucleic acid or promote cell apoptosis; if the transcription, expression, activity or transportation to nucleus of acetylcholinesterase or its fragment or variant in the test group is statistically reduced (preferably significantly reduced transcription, expression, or activity, such as reduced by more than 20%, preferably reduced by 50%; more preferably reduced by greater than 80%), it suggests that the candidate materials are potential materials to protect nucleic acid or inhibit cell apoptosis.

In another preferred embodiment, said candidate materials include (but not limited to): interfering molecules, nucleic acid inhibitors, binding molecules (e.g., antibody or ligand), small molecules compound designed for acetylcholinesterase or the fragment or variant or encoding gene thereof; recombinant plasmids expressing acetylcholinesterase or the fragment or variant thereof; or fusion proteins of acetylcholinesterase or the fragment or variant thereof with nuclear importer, or the encoding gene thereof, etc.

In another preferred embodiment, said system is selected from: cell system (such as cells expressing acetylcholinesterase or its fragment or variant) (or cell culture system), subcellular system, solution system, tissue system, organ system or animal system.

In another preferred embodiment, said method also includes: performing further cell experiments and/or animal tests on obtained potential materials, to further select and determine materials useful for nucleic acid stability or cell apoptosis.

In another aspect of the present invention, provided is a method for screening potential drugs which can inhibit cell apoptosis or nucleic acid stability, wherein the method comprises:
(1) in a test group, treating cells expressing or containing acetylcholinesterase or the fragment or variant thereof with candidate materials; and
(2) detecting intracellular DNA breakage in said test group, and comparing with that of a control group, wherein said control group is the cells expressing or containing acetylcholinesterase or the fragment or variant thereof without adding said candidate materials;
   wherein, if DNA breakage in said test group significantly increases compared with the control group (preferably significantly increases, such as increases by more than 20%, preferably increases by 50%; more preferably increases by greater than 80%), it suggests that the candidate materials are potential drugs to inhibit cell apoptosis or nucleic acid stability.

In another preferred embodiment, said cell is a fixed cell; and / or said cell is a cell with membrane treated for permeabilization.

In another preferred embodiment, fixation or permeabilization treatment of said cell will not result in DNA breakage.

In another preferred embodiment, said method further includes: performing further cell experiments and / or animal tests on the obtained potential materials, to further select and determine materials useful for nucleic acid stability or cell apoptosis.

In another preferred embodiment, said acetylcholinesterase or the fragment or variant thereof is an acetylcholinesterase fragment without an esterase active site (e.g., position 234 or an adjacent position thereof, position 365 or an adjacent position thereof and/or position 478 or an adjacent position thererof in the acetylcholinesterase of human origin).

In another preferred embodiment, said acetylcholinesterase is an N-terminal fragment of acetylcholinesterase, such as position 32-138, position 2-138, position 33-138, position 2-191, etc.

In another aspect of the present invention, provided is a method for screening potential drugs which can regulate cell apoptosis or nucleic acid stability, wherein the method comprises:
(1') treating a system (e.g., in vitro system, solution system) with candidate materials, wherein said system contains acetylcholinesterase or acetylcholinesterase fragment or variant as well as a nucleic acid (e.g., DNA, or DNA-containing plasmid); and
(2') detecting nucleic acid breakage within said system; if nucleic acid breakage increases (preferably significantly increases, such as increases by more than 20%, preferably increases by 50%; more preferably increases by greater than 80%), it suggests that the candidate materials are potential materials to damage nucleic acid or promote cell apoptosis; if nucleic acid breakage decreases (preferably significantly decreases, such as decreases by more than 20%, preferably by decreases 50%; more preferably decreases by greater than 80%), it suggests that the candidate materials are potential materials to protect nucleic acid or inhibit cell apoptosis.

In another preferred embodiment, step (1') includes: in test group, treating said system with candidate materials; step (2') includes: detecting nucleic acid breakage in said test group, and comparing with that of a control group, wherein said control group is the system containing acetylcholinesterase or the fragment or variant thererof as well as nucleic acid without adding said candidate materials; if nucleic acid breakage increases, it suggests that the candidate materials are potential materials to damage nucleic acid or promote cell apoptosis; if nucleic acid breakage decreases, it suggests that the candidate materials are potential materials to protect nucleic acid or inhibit cell apoptosis.

Due to the disclosure, other aspects of the present invention are obvious to those skilled in the art.

### DESCRIPTION OF THE DRAWINGS

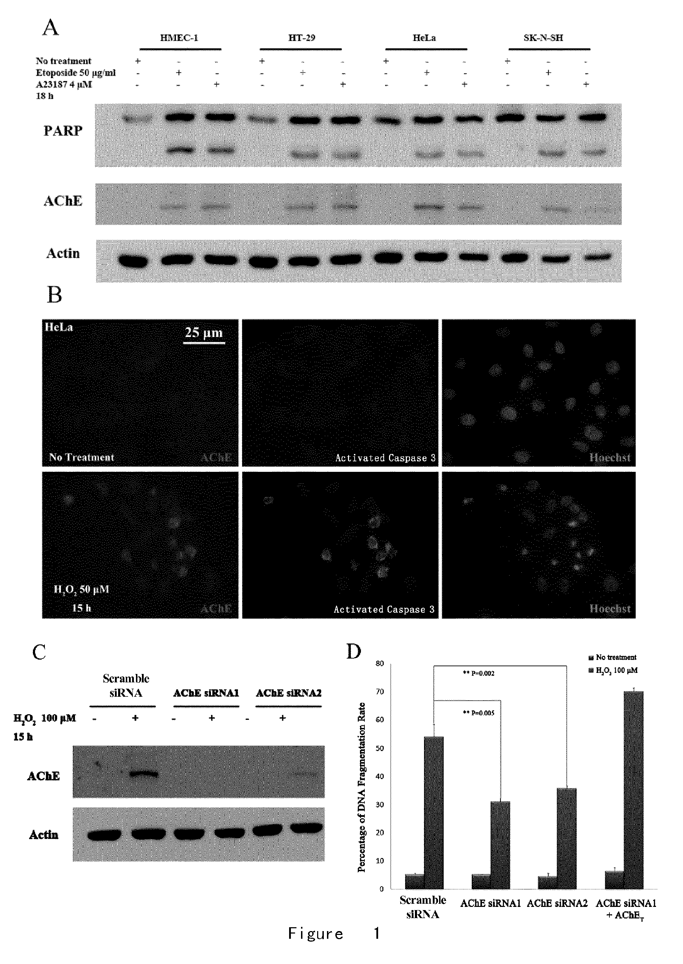

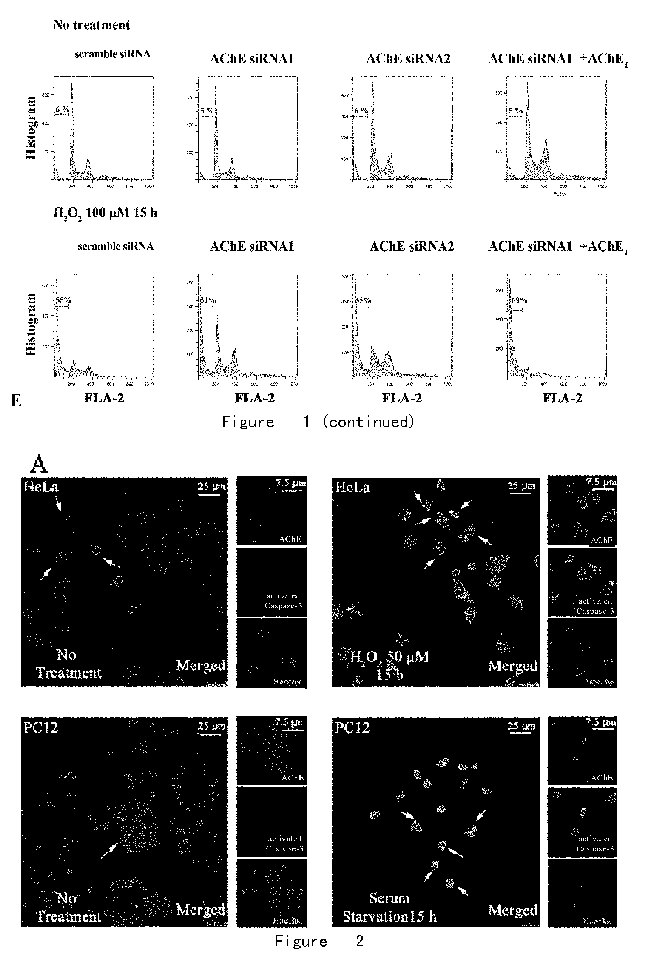

**Figure 1****. Different methods for inducing AChE expression in different cells.**
   A. 4 cell lines from different origins were treated in Etoposide (50 µg/ml) or A23187 (4µM) for 18h, after which cell lysates were subject to immunoblot assay with anti-AChE and anti-PARP antibodies, with the expression of Actin as a control.
   B. HeLa cells were treated or not treated with H₂O₂ for 15h, and cells were subject to immunofluorescence staining; AChE protein and cleaved caspase 3 were observed and nucleuses were stained with Hoechst dye.
   C. HeLa cells were transiently transfected with siRNA1 and siRNA2, respectively, after 24h, treated by H₂O₂ for 15h, and AChE expression in cell lysates was dertermined. Cells transiently transfected with unrelated siRNA (Scramble, random composition provided by synthesis company) were used as control.
   D. HeLa cells were transfected with siRNA1, siRNA2, Scramble, respectively, or co-transfected with siRNA1+AChE expression plasmid (plasmid encoding human S-AChE protein), and after 24h, the apoptosis of HeLa cells was analyzed, and the number of DNA fragments was calculated. Data were mean ± SD from triplicate assay. ***P*<0.01.
   E. Representative Histogram of D.

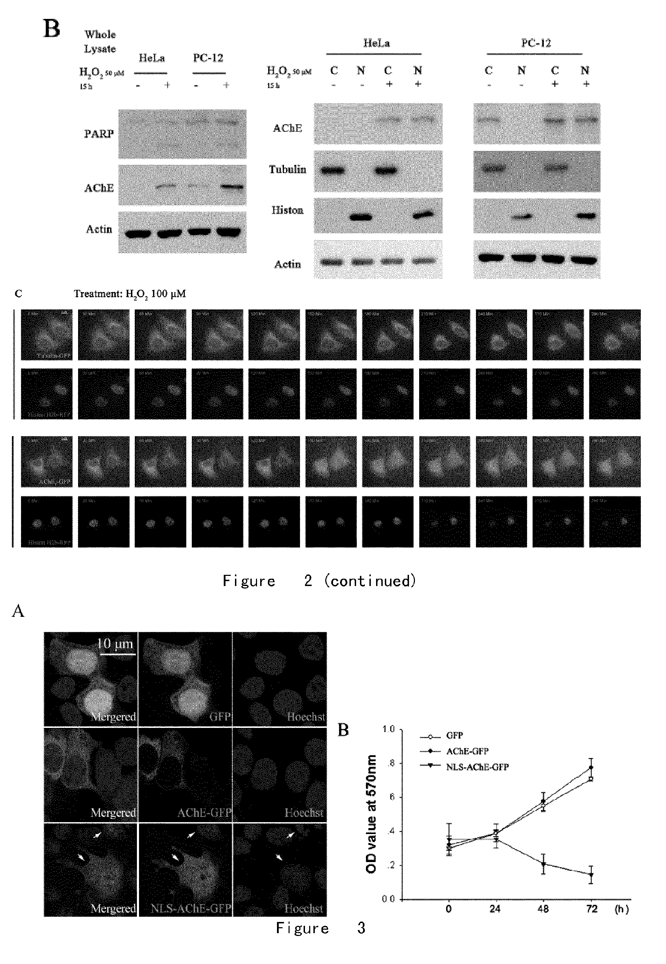

**Figure 2****. The subcellular distribution of AChE in normal or apoptotic cells.**
   A. Confocal microscopy images of HeLa cells which grew normally or treated by H₂O₂ for 15h, and confocal microscopy images of PC-12 cells which grew normally or treated by serum starvation for 15h. In order to show the details, positions shown by white arrows were magnified on the right side of each figure.
   B. Various treated cells described in A were subject to cytoplasm-nucleus separation, before being analyzed by immunoblotting. Cell nucleus (N) and cytoplasm (C) fragments were identified with anti- Histone H3 antibody and anti-α-tubulin antibody.
   C. AChE-GFP-encoding plasmid and Histone H2b-RFP-encoding plasmid were co-transfected into HeLa cells, before inducing cell apoptosis with 100 µM H₂O₂, and the change in position of AChE after cell apoptosis initiation was traced and photographed by a living cell real-time imaging system.

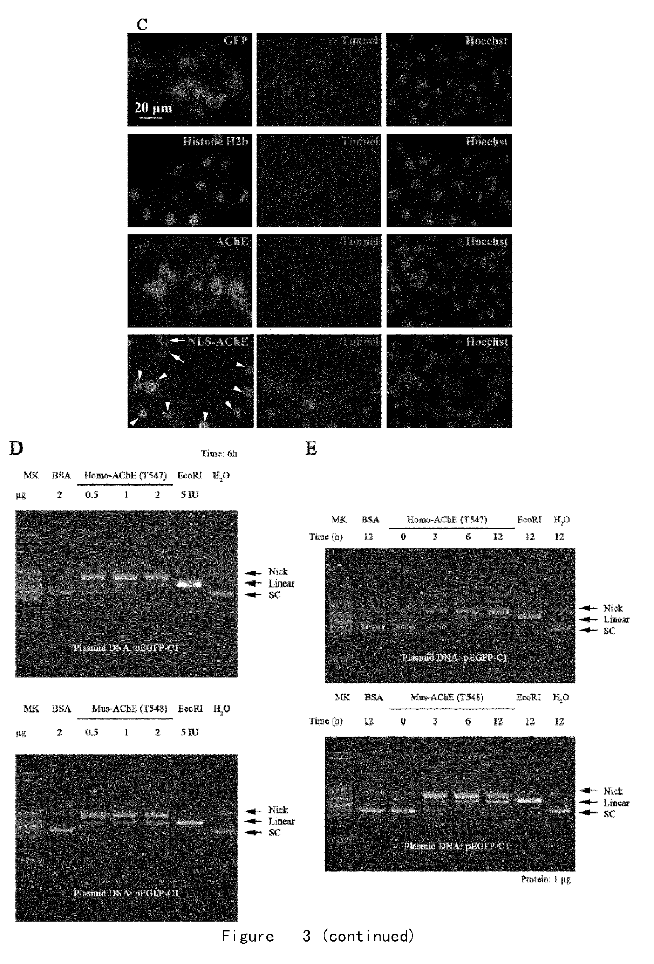

**Figure 3****. The expression of AChE within nucleus initiated the apoptosis of HeLa cells, and AChE cleaved super coiled (SC) plasmid DNA into Nick and Linear DNA.**
   A. Nuclear localization signal (NLS) guided AChE from cytoplasm to nucleus. Plasmids encoding GFP, AChE-GFP and NLS-AChE-GFP were transiently transfected into HeLa cells, and observed under laser scanning confocal microscope. GFP and NLS-AChE-GFP were localized in both cytoplasm and nucleus, whereas AChE-GFP was only distributed in cytoplasm.
   B. Each group of cells in A was enriched by FACS screening. GFP positive cells were transfected to the 96-well plate, and MTT assay was performed at indicated time points. 2 independent assays were performed, and data were shown as mean ± SD.
   C. HeLa cells which were transfected by plasmids encoding GFP, Histone H2b-GFP, AChE-GFP and NLS-AChE-GFP were stained by TUNEL. GFP and Histone-GFP were used as controls for nuclei-located protein.
   D. The results of 1% agarose electrophoresis before which pEGFP-C1 plasmid DNA and Homo or Mus AChE protein (0, 0.5, 1 and 2 µg) were coincubated for 6h. EcoR was used to indicate linear DNA. MK indicated molecular weight marker.
   E. the plasmid splitting with increasing time of the incubation of pEGFP-C1 plasmid DNA (150 ng) and Homo or Mus AChE protein (1 µg)). The above experiment was repeated for 3 times.

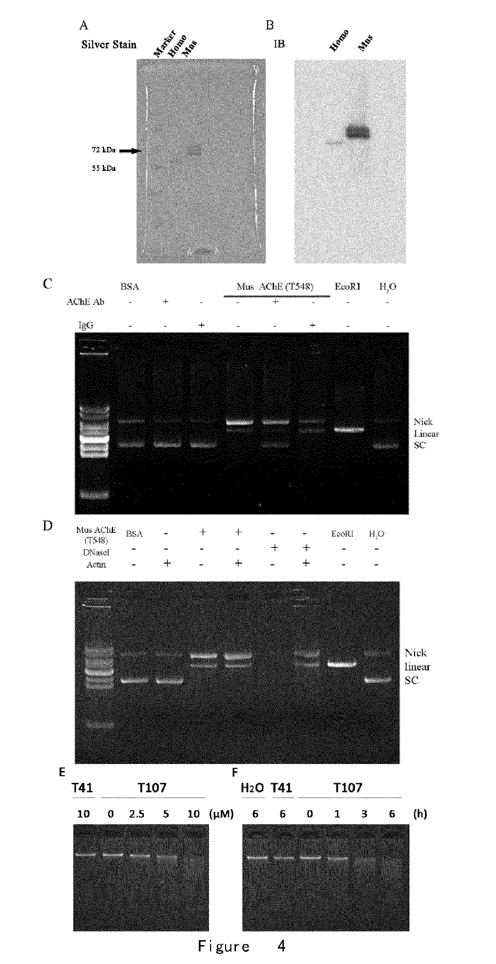

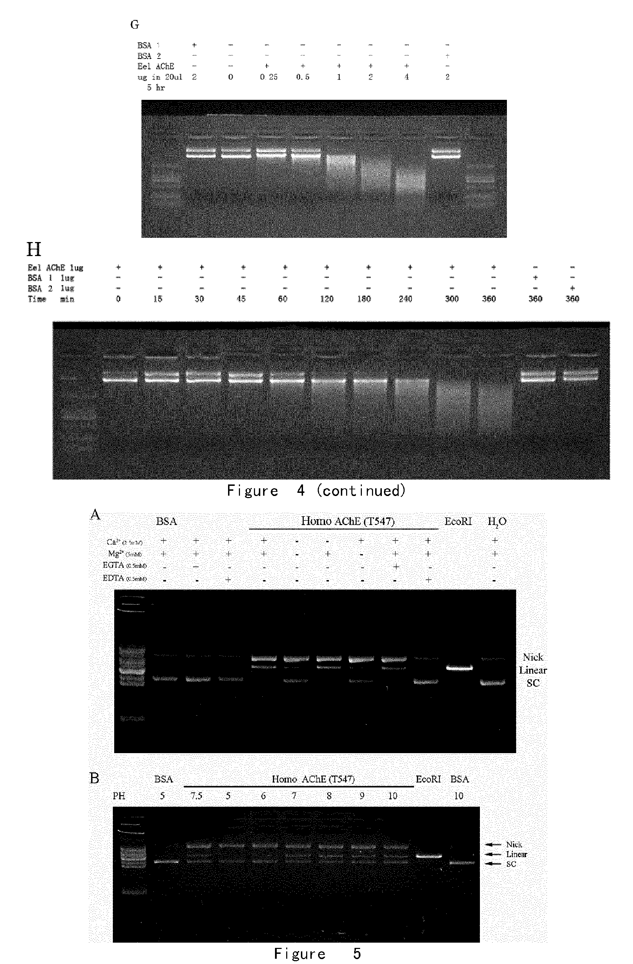

**Figure 4****. The identification of nuclease activity of AChE.**
   A. Silver staining analysis of purified Homo and Mus AChE proteins.
   B. Immunoblotting of purified Homo and Mus AChE proteins.
   C. Removing Mus AChE protein prevented *in vitro* DNA cleavage. Protein G glass beads conjugated with AChE antibody or rabbit IgG were added to the *in vitro* DNA cleavage system, and after 30 minutes, centrifuged to take the supernatant, before adding the substrate pEGFP-C1 plasmid DNA and incubating at 37□ for 6 h. Rabbit IgG was used as an iso-specific control. The result represents the outcome of 3 independent experiments.
   D. The results of electrophoresis after the co-incubation of pEGFP-C1 plasmid DNA and Mus AChE protein and DNase I. G-Actin, an inhibitor of DNase I, was added before the addition of the substrate pEGFP-C1 plasmid DNA.
   E. The 32-72AA fragment (T41) and 32-138AA fragment (T107) of Homo AChE were artificially synthesized, and the peptide fragments were incubated in different doses with mice genome DNA for 6h, and then subjected to DNA gel electrophoresis.
   F. Artificially synthesized the 32-72AA fragment (T41) and 32-138AA fragment (T107) of Homo AChE, the peptide fragments were incubated with mice genome DNA for different time, then subject to DNA gel electrophoresis.
   G. Commercial AChE isolated from Eel was incubated in different doses with plasmid DNA, and two bovine serum albumins (BSA1, BSA2) purchased from different companies were used as control at the same time; after being incubated for 5h, they were subjected to DNA gel electrophoresis.
   H. Commercial AChE isolated from Eel was incubated with plasmid DNA for different durations, and two bovine serum albumins (BSA1, BSA2) purchased from different companies were used as control at the same time. They were then subjected to DNA gel electrophoresis.
**Figure 5****. Biochemical condition analysis of DNase activity of AChE.**
   A. In the presence of 2.5 mM CaCl₂, 5 mM MgCl₂, 0.5 mM EGTA, 0.5 mM EDTA or the combination thereof, plasmid DNA (pEGFP-C1 plasmid) was incubated with Homo AChE protein for 6h.
   B. The influence of pH value on AChE nuclease activity. Plasmid DNA was incubated with Homo AChE protein under different pH conditions for 3h.

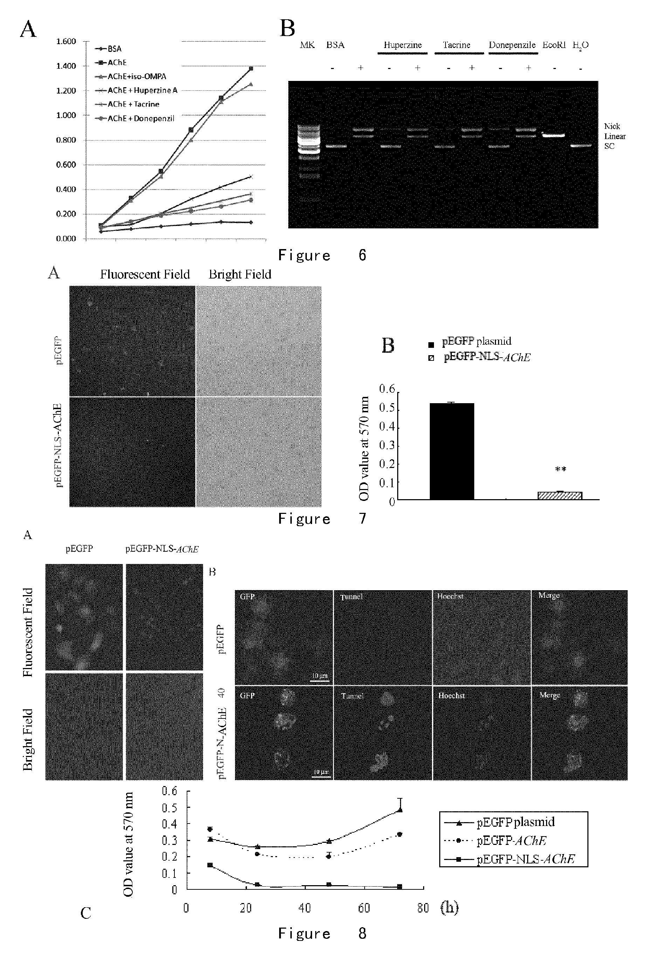

**Figure 6****. AChE nuclease activity was not affected by the inhibition of AChE esterase activity, and the structural analysis of DNA binding sites of AChE protein.**
   A. AChE activity was determined by Ellman's assay. 3 AChE inhibitors (10 µM Huperzine A, 1 µM Tacrine and 5 µg/ml Donepenzile) were added to determine the presence of inhibition. Iso-OMPA (the inhibitor of BChE, purchased from Sigma) was used as a negative control.
   B. Plasmid DNA was co-incubated with Mus AChE protein, and the inhibitor of AChE was added simultaneously or not. The inhibitor of AChE was added 30min before the addition of the substrate plasmid DNA. (-) represents no addition of AChE, whereas (+) represents the addition of AChE.
**Figure 7****. *NLS-AChES* gene (*NLS-AChES*) induced the apoptosis of colon cancer SW620 cells (×100).**
   A. After 72h of flow-cytometrically screening, the *NLS-AChES* transfection positive cell ratio and cell morphological changes shown by inverted fluorescence microscope.
   B. The cell death rate promoted by *NLS-AChES* determined by MTT after 72h of flow-cytometrically screening (n=2, *p*<0.001).
**Figure 8****. *NLS-AChES* gene significantly induced the apoptosis of cervical cancer HeLa cells.**
   A. The positive cell ratio of flow cytometer screening shown by an inverted fluorescence microscope and *NLS-AChES* gene induced cells to present morphological features of apoptosis (x200).
   B. Tunnel staining shows that the way *NLS-AChES* gene induced cell death was apoptosis.
   C. MTT determined that within 24h-72h after flow-cytometrically screening, *NLS-AChES* gene promoted cell death compared with EGFP gene and *AChES* gene, suggesting that AChES protein played a role of promoting apoptosis after transportation to nucleus (n=2).

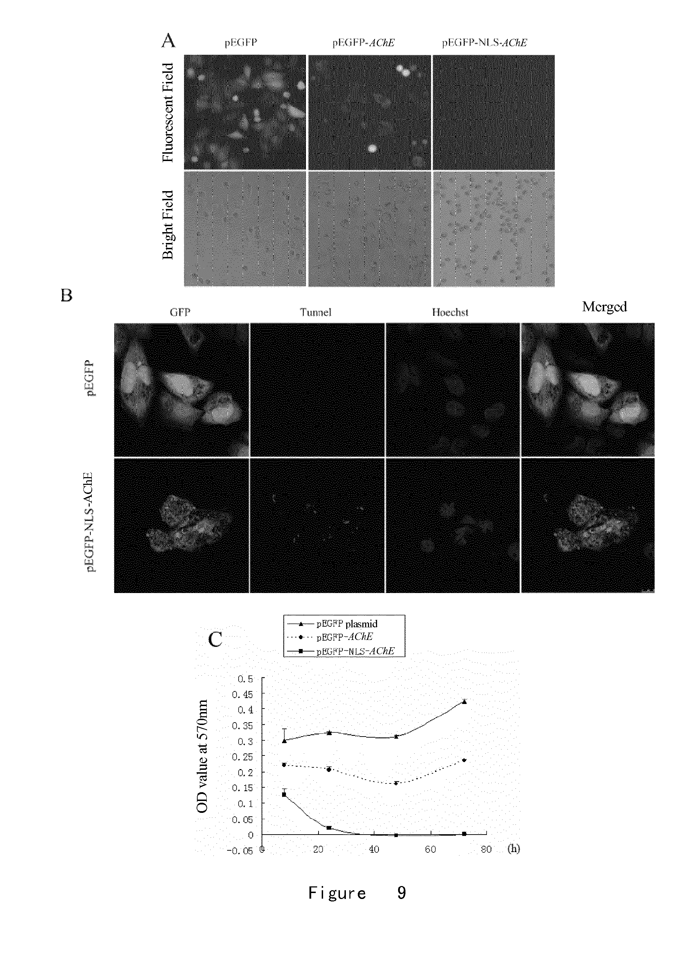

**Figure 9****. *NLS-AChES* gene significantly induced the apoptosis of liver cancer BEL7404 cells**
   A. Positive cell ratio of flow cytometer screening was shown by an inverted fluorescence microscope and *NLS-AChES* gene induced cells to present \morphological features of apoptosis (x200).
   B. Tunnel staining shows that the way *NLS-AChES* gene induced cell death was apoptosis.
   C. MTT determined that within 24h-72h after flow-cytometrically screening, *NLS-AChES* gene promoted cell death compared with EGFP gene and *AChES* gene, suggesting that AChES protein played a role of promoting apoptosis after transportation to nucleus (n=2).

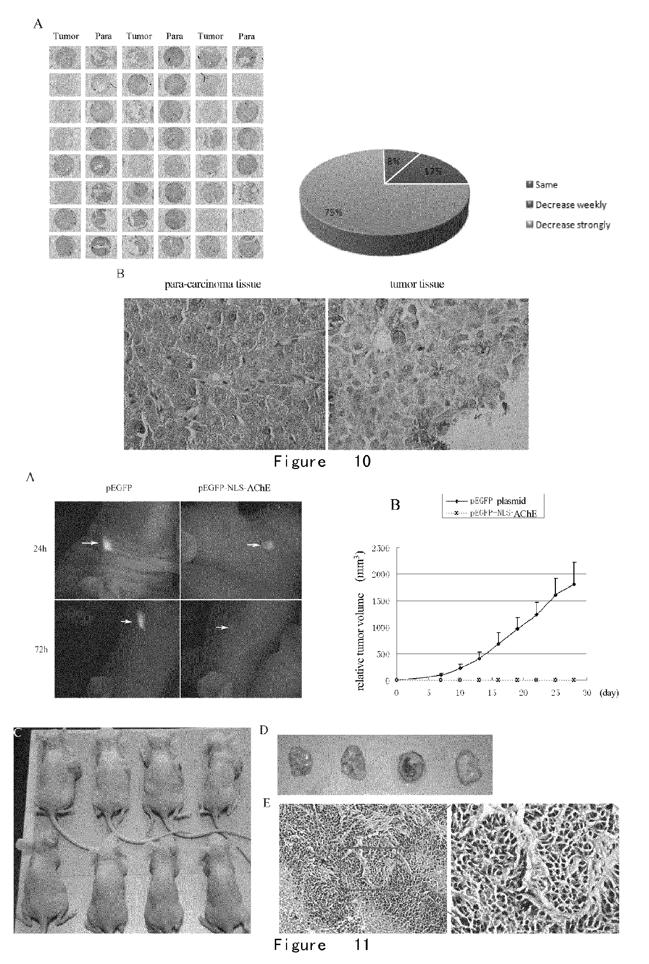

**Figure 10****. Immunohistochemistry assay showed the expression of AChE protein decreased in clinical liver cancer tissue samples.**
   A. Left panel, immunohistochemical results of tissue chip AChE proteins (x40); Right panel, analytical and statistical results of the left panel.
   B. Enlarged figure of pericarcinomatous and corresponding cancer tissue samples (x600).
**Figure 11****. *NLS-AChES* gene inhibited BEL-7404 liver cancer cells to form tumor subcutaneously in nude mice.**
   A. The presence of BEL-7404 cells transfected with pEGFP or *pEGFP-NLS-AChES* plasmid at 24hr, 72hr after flow cytometry screening determined by stereo fluorescence microscope.
   B. Cells were implanted subcutaneously in nude mice after flow-cytometrically screening, and the curve of tumor volume change in 28 days was shown.
   C. Tumor formation in day 28 was shown by photo of nude mice.
      Row 1, implanted pEGFP cells, tumor significantly formed (the third, fourth from the right, tumor was too large to be torn);
      Row 2, implanted *pEGFP-NLS-AChES* cells, tumor not formed.
   D. Tumors taken from pEGFP group in vivo in day 28.
   E. the characteristics of tumor tissue cell shown by paraffin section-HE staining (left panel×100; right panel×600).

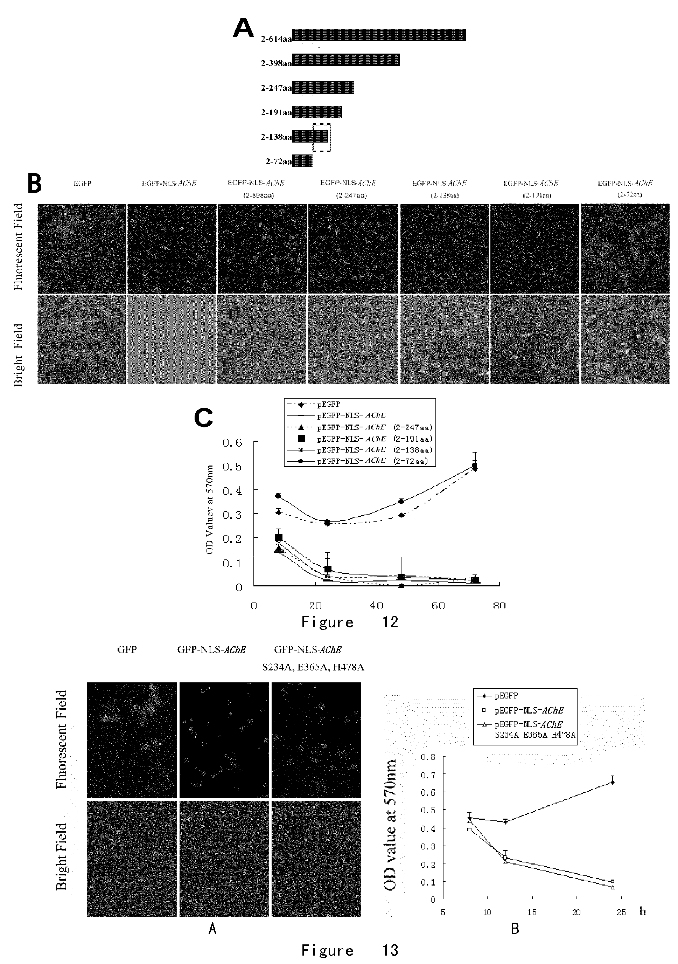

**Figure 12****. Identificaton of the fragment with apoptosis-promoting function in AChE protein molecule.**
   A. Schematic plot of deletion mutation in AChE protein molecule.
   B. Each mutant plasmid shown in A was transfected to HeLa cells, respectively, and the transfection positive cells were sorted through flow cytometer, and then implanted to 96-well plate; and the change in cell morphology was shown at 72 h by a fluorescent inverted phase contrast microscope (×200). As can be seen, 2-138AA fragment or a longer fragment both provided the cells with death features; whereas 2-72AA fragment had no significant influence on cell survival.
   C. Cells from B were subjected to MTT assay, and a curve of cell growth curve was plotted. 2-138AA fragment is provided with apoptosis-promoting function of a full-length AChE, while 2-72AA fragment is deprived of this function completely.
**Figure 13****. AChE acetylcholinesterase active site is not relative to apoptosis-promoting function of AChE protein.**
   A. After *pEGFP-NLS-AChE* (S234A, E365A, H478A) esterase active site-mutated plasmid was transfected to cervical cancer HeLa cells, observation of cell morphological changes by fluorescence microscope found that *pEGFP-NLS-AChE* (S234A, E365A, H478A) plasmid significantly promoted cell apoptosis compared with empty plasmid (pEGFP).
   B. The result of MTT assay showed that *pEGFP-NLS-AChE* (S234A, E365A, H478A) plasmid induced cell apoptosis to the similar extent with wild type plasmid *pEGFP-NLS-AChE.* These data suggests that AChE acetylcholinesterase active site is not relative to apoptosis-promoting function of AChE protein.

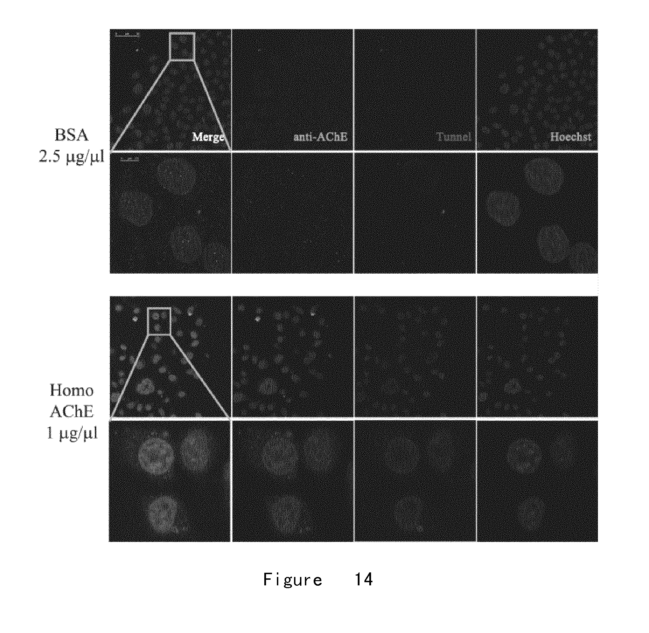

**Figure 14****.** DNA breakage within cell of AChE protein group compared with BSA control group in HeLa cells is provided, from which it can be seen that DNA within cell of AChE protein group presented obvious breakage.

### DETAILED DESCRIPTION

After in-depth research of acetylcholinesterase (AChE), the inventors have found that AChE has new use in regulating the stability of nucleic acid and cell apoptosis. Based on the above new discovery, a series of formulations with applicative value can be developed, such as agents promoting or inhibiting cell apoptosis, agents inhibiting tumor, agents protecting nucleic acid from damage. Furthermore, AChE can be used as a target to screen a series of of agents which may regulate the expression or activity of AChE in order to play a role of regulating the stability of nucleic acid or cell apoptosis. Also, the present invention first discloses that AChE can be a novel tumor "killer" after being combined with nuclear importer.

### Terms

As used herein, the "nuclease" refers to an enzyme causing damage on nucleic acid, and said damage is, for example: cleavage, degradation. Said "nuclease" acts on the middle or the head and tail of nucleic acid strand, making nucleic acid rupture, notch or become linear. Said "nuclease" is preferable a deoxyribonuclease.

As used herein, the "nucleic acid" includes deoxyribonucleic acid, ribonucleic acid, with A, T (or U), C or G as basic component units. Said nucleic acid includes an unmodified form or a form with parts of sites modified.

As used herein, said "nuclear importer" refers to a targeting material, which has a targeting effect on cell nucleus, and is a general designation of material which can be mutually connected with guided material (such as AChE) by means of covalent linkage (e.g., fusion), coupling, attachment, encapsulation, embedment and other ways, and then can be directed guided material (such as AChE) into cell nucleus.

As used herein, said "tumor" is various benign or malignant tumors, nasopharyngeal carcinoma, esophagus cancer, gastric carcinoma, liver cancer, breast cancer, colorectal cancer, prostate cancer, lung cancer, cervical cancer, leukemia, oral cancer, salivary gland tumor, nasal cavity and paranasal sinuses malignant cancer, laryngeal cancer, ear cancer, eye cancer, thyroid cancer, mediastinal tumor, chest wall, pleural cancer, intestinal cancer, bile duct cancer, pancreatic and ampullary tumor, mesenteric and retroperitoneal tumor, kidney tumors, adrenal tumor, bladder cancer, prostate cancer, testicular cancer, penile cancer, endometrial cancer, ovarian cancer, malignant trophoblastic tumor, vulvar and vaginal cancer, malignant lymphoma, multiple myeloma, soft tissue tumors, bone tumors, skin tumors and accessories, malignant melanoma, nervous system tumors, pediatric tumor.

As used herein, the "comprise", "possess/have" or "include" emcompass "contain", "consist mainly of", "consist essentially of" and "consist of"; "consist mainly of", "consist essentially of" and "consist of" belong to the hypogyny conception of "comprise", "possess" or "include".

Said "comprise", "possess/have" or "include" amino acid sequence of X polypeptide (or Y nucleotide sequence), when used for sequence, are intented to define limited situations; in addition to core "amino acid sequence of X polypeptide (or Y nucleotide sequence)", to both or either of the ends only limited amino acids (or nucleotides) can be added, for example 1-600, preferably 1-500, more preferably 1-400, more preferably 1-300, more preferably 1-200, more preferably 1-100, more preferably 1-50, more preferably 1-30, more preferably 1-20, more preferably 1-10, more preferably 1-5 amino acids can be added. Or for example 1-1800, preferably 1-1500, more preferably 1-1200, more preferably 1-900, more preferably 1-600, more preferably 1-300, more preferably 1-150, more preferably 1-90, more preferably 1-60, more preferably 1-30, more preferably 1-15 nucleotides, more preferably 1-5 nucleotides can be added.

As used herein, the "operably linked to" or "operably connected to" refers to a situation that certain parts of a linear DNA sequence can regulate or control the activity of other parts of the same linear DNA sequence. For example, if a promoter controls the transcription of sequence, it is operably linked to the coding sequence.

As used herein, said "protein transduction domain polypeptide" refers to a protein having protein delivery function, which can be linked or coupled with target protein and deliver the target protein into cell.

As used herein, said "protein transduction" refers to a method of protein transduction of target protein (e.g., acetylcholinesterase) using recombinant technology or chemical coupling etc., which means that the target protein is linked to protein transduction domains (PTDs) using recombinant technology or chemical coupling etc., and then the target protein is delivered into a cell directly, e.g., under the action of PTD. Protein transduction domains which have already been found include TAT, Antp and VP22 etc. (Beerens A M J ,A1 Hadithy A F Y, Rots M G, et al. Protein transduction domain and their utility in gene therapy. Curr Gene Ther, 2003, 3 (5) :486 ~ 494).

As used herein, said "nuclear localization signal" refers to a polypeptide having nuclear localization function, which can be linked to or coupled with a target protein, and then the target protein is delivered to cell nucleus. Said "nuclear localization signal" is any molecules which can import a directable protein known in the art into cell nucleus, as long as it has the nuclear localization function. Among which, one kind of nuclear localization signal comprises core sequence, and said core sequence consists of polypeptide having 3-4 lysines or arginines (such as hexapeptide), with a basic structure of Lys-Arg/Lys-X-Arg/Lys, having no acidic amino acids or amino acids of macromolecule; proline or glycine can be lateral to core NLS. Said "nuclear localization signal" can be a protein only containing core sequence, and can be a protein containing longer sequence of other sequences as well.

### Acetylcholinesterase

Acetylcholinesterase (AChE) is an enzyme well known to those skilled in the art in the prior art, and its well-known function in the past is as an important molecule to hydrolyze neurotransmitter acetylcholine in cholinergic nerve synapse and neuromuscular junction, and in addition, it is also involved in some "nonclassical functions", e.g., playing a role in neuritogenesis, cell proliferation, cell adhesion, synaptogenesis, amyloid fibril aggregation, hematopoiesis and thrombopoiesis process. The present invention discloses for the first time that it plays a role in regulating the stability of nucleic acid, i.e., it can be used as a nuclease.

Unexpectedly, the inventors found from extensive research that in addition to the activity of hydrolyzing ACh, AChE had nuclease activity and it participated in chromosomal DNA degradation in cell apoptosis process. After a cell initiates apoptosis signal, the expression amount of AChE is increased and transported from cytoplasm to nuclear. In vitro experiments found that isolated and purified AChE proteins from human and mice origins were all capable of cleaving plasmid DNA with superhelical structure into DNA with nicked and linear structure. More importantly, AChE expression in nucleus of a tumor cell would cause cell death.

In the present invention, said AChE protein (polypeptide) can be naturally occurring, for example, is can be isolated or purified from animals and plants. Furthermore, said AChE protein can be artificially prepared; for example, recombinant AChE protein can be produced according to conventional genetic engineering recombinant technologies. Preferably, recombinant AChE protein can be used.

According to the present invention, AChE originating from mammal including human and mice, and AChE originating from fish (Eel), both lead to the degradation of genome DNA and plasmid DNA, i.e., both have nuclease activity. Therefore, it should be understood that a suitable AChE from any origin can be used in the present invention and fall within the scope of the claims, and they are not only limited to AChE from origins of individual species.

Any suitable AChE protein can be used in the present invention. Said AChE protein includes full-length AChE protein or the bioactive fragment (or called active fragment) thereof. For example, the amino acid sequence of said AChE protein can be essentially the same as that of SEQ ID NO: 5 (or GenBank Accession No. P22303.1, or GenBank Accession No. NP_033729.1, or other already known AChE proteins from different animal and plant origins).

As a preferred embodiment of the present invention, bioactive fragments of said AChE protein are selected from: (b) a peptide of aa 32-138 of SEQ ID NO: 5; (c) a peptide of aa 2-138 of SEQ ID NO: 5; (d) a peptide of aa 2-191 of SEQ ID NO: 5; (e) a peptide of aa 2-247 of SEQ ID NO: 5; (f) a peptide of aa 2-398 of SEQ ID NO: 5; (g) a peptide of aa 32-578 of SEQ ID NO: 5; (h) a peptide of aa 32-579 of GenBank Accession No. NP_033729.1; (i) a polypeptide of amino acid sequence of any of (e)-(g), in which variations occur in position 234, 365 and/or 478 (such as S234A, E365A, H478A); (j) a polypeptide formed by amino acid sequence of polypeptide from any of (b)-(i) (i.e., selected from any of (b),(c),(d),(e),(f),(g),(h) or (i)) with one or more (e.g., 1-20; preferably 1-15; more preferably 1-10; more preferably 1-5; more preferably 1-3) amino acid residue substitutions, deletions or additions, and has the function of the polypeptide of SEQ ID NO: 5; (k) fragments of any of (b)- (j) (i.e. selected from any of (b),(c),(d),(e),(f),(g),(h),(i) or (j)) polypeptide having the function of the polypeptide of SEQ ID NO: 5 (preferably, sharing greater than 20% sequence identity with SEQ ID NO: 5; more preferably greater than 30%; more preferably greater than 40%; more preferably greater than 50%; more preferably greater than 60%; more preferably greater than 70%; more preferably greater than 80%; more preferably greater than 85%; more preferably greater than 90%; more preferably greater than 95%; more preferably greater than 98% or 99% of sequence identity with SEQ ID NO: 5); (1) a polypeptide having the function of the polypeptide of SEQ ID NO: 5 and containing amino acid sequence of any (b)-(k) polypeptide. The bioactive fragments of the AChE protein described above retain the biological activity of full-length AChE protein, and the acetylcholinesterase active site-mutant also completely retains the apoptosis-promoting biological function of its wide type protein.

aa 1-31 from N-end of AChE protein is signal peptide sequence, which is known in the prior art, as reported by J Neural Transm (2009) 116:1435-1442; Pro-apoptotic protein-protein interactions of the extended N-AChE terminus or THE JOURNAL OF BIOLOGICAL CHEMISTRY; Vol. 279, No. 28, Issue of July 9, pp. 29740-29751, 2004. Signal peptide sequence plays a role of protein guidance, but generally is not the necessary structure for exerting protein activity. Therefore, combing with the results of the present invention, it can be determined that position 32-138 of AChE protein can play a role of nuclease.

The amino acid sequences of AChE protein or the bioactive fragments thereof formed through one or more amino acid residue substitutions, deletions or additions were also included in the present invention. AChE protein or the bioactive fragments thereof include substituted sequences for a part of conserved amino acids, and said amino acid-substituted sequences have no effect on its activity or retain part of its activity. Suitably substituting amino acids is a well-known technique in the art, and said technique can be easily practised without changing biological activity of the obtained molecule. These techniques make those skilled in the art recognize that, generally, altering single amino acid in non-essential region of a polypeptide essentially brings no change to biological activity. See Watson et al., Molecular Biology of The Gene, 4th edition, 1987, The Benjamin/Cummings Pub. Co. P224.

Bioactive fragments of any AChE protein can be used in the present invention. Herein, the meaning of bioactive fragments of AChE protein is that as a polypeptide, it still retains full or partial function of full-length AChE protein. Most often, said bioactive fragments retain at least 50% of activity of full-length AChE protein. Under more preferred conditions, said bioactive fragments can retain 60%, 70%, 80%, 90%, 95%, 99%, or 100% of activity of full-length AChE protein.

The present invention may also employ a modified or improved AChE protein, for example, may employ a modified or improved AChE protein to improve its half-life, effectiveness, metabolism and/or protein efficacy. Said modified or improved AChE protein may be a conjugate of AChE protein, or it can comprise substituted or artificial amino acids. Said modified or improved AChE protein may share less common points with naturally occurring AChE protein, but it can also play a role of nuclease, and brings no further adverse effects or toxicity. That is, any variation form not affecting biological activity of AChE protein can be used in the present invention.

It's well known that AChE protein is present in many animals and plants with high conservation, e.g., in human and mice, and the sequence identity (homology) of AChE protein reaches 88%. Therefore, it should be understood that all AChE from different animal and plant origins are included in the present invention. Preferably, in comparison with amino acid sequences of SEQ ID NO: 5 or the bioactive fragments thereof, their sequence identity is higher than 60%, more preferably higher than 70%, more preferably higher than 80%, more preferably higher than 85%, more preferably higher than 88%, more preferably higher than 90%, more preferably higher than 95%, more preferably higher than 98%.

In mammals, AChE protein is encoded by one gene, but there exists three subtypes due to different C-terminal splicing form, that is S form of acetylcholinesterase (AChES), R form of acetylcholinesterase (AChER) and H form of acetylcholinesterase (AChEH), respectively. These three subtypes all retain classical function inherent in AChE, i.e., hydrolyzing acetylcholine in synapse and neuromuscular junction of peripheral nervous system, blocking nerve impulse transmission and so on. AChER and AChEH also have sequence at position 1-547 (containing nuclease functional site) same as AChES, and they are thus all included in the present invention.

Once said protein sequence is isolated and obtained, the protein can be obtained in large quantities by recombinant method. They are obtained generally by cloning its coding gene into a vector, before transforming the vector into a cell, and then isolating from host cells after proliferation through conventional methods. Furthermore, for shorter proteins, artificial synthesis (e.g., synthesized by peptide synthesizer) methods can be used as well to synthesize relevant sequences, and artificial synthesis method can result in desired proteins easily and rapidly.

The present invention also includes isolated nucleic acid encoding bioactive fragments of said AChE protein, which can also be its complementary strand. DNA sequences encoding bioactive fragments of AChE protein can be artificially synthesized for complete sequence, and can be obtained by PCR amplification method as well. After obtaining DNA sequences encoding bioactive fragments of said AChE protein, they are combined into a suitable expression vector, and transformed to suitable host cell. Finally, the desired protein can be obtained by culturing the transformed host cell before isolating and purification.

Based on the new discovery of the inventors, the present invention provides use of AChE protein or the bioactive fragments thereof, including but not limited to: as a nuclease (can be used for in vitro therapeutic application), for preparing a composition regulating the stability of nucleic acid (e.g., DNA), for preparing a composition regulating cell apoptosis, for screening a material inhibiting and regulating the stability of nucleic acid, or for screening a material regulating cell apoptosis, and so on.

AChE protein or its agonists have significant effect on damaging nucleic acid and/or promoting cell apoptosis, which is of high application value. Tumor is a disease caused by excessive proliferation of malignant cells, and AChE protein or its agonists (particularly, materials guiding AChE transport to nucleus) can be used to damage nucleic acids of tumor cells, in order to achieve the effect of killing tumor cells efficiently. Examples of the present invention have demonstrated that the fusion protein of AChE and nuclear localization signal has excellent tumor-killing effect.

Said AChE protein itself can be directly used to promote cell apoptosis, AChE protein is generally a protein with a molecular weight less than approximately 70kD; while its fragment has a smaller molecular weight. It's known in the art that the protein importation to the nucleus is through diffusion or transport, and whether it can be transported to the nucleus relates to the size of protein molecule, wherein a protein having smaller molecular weight or a diameter less than 10 nm can pass through nuclear pore complex (NPC) into the nucleus by diffusion (see Virol Sin. 2010 Apr; 25(2): 79-85. Epub 2010 Apr 9; The nucleocytoplasmic transport of viral proteins), to play a role in DNA degradation. Furthermore, AChE protein can be combined with materials to be transported into nucleus to enter cell nucleus more effectively.

### Regulator

As used herein, said AChE agonists include stabilizer, accelerator, up-regulator etc. Any material enhancing the activity of AChE protein, maintaining the stability of AChE protein, promote the expression of AChE protein, prolonging effective action time of AChE protein, improving AChE gene transcription and translation can be used in the present invention, as an effective material used for damaging nucleic acid and/or promoting cell apoptosis. AChE agonists further include some nuclear importers which can direct AChE into nucleus, or ions providing relatively good environment for AChE action, such as magnesium ions.

As used herein, said AChE antagonists include inhibitor, down-regulator, retardant, blocker etc. Any material reducing the activity of AChE protein, decreasing the stability of AChE protein, inhibiting the expression of AChE protein, reducing effective action time of AChE protein or inhibiting AChE transcription and translation can be used in the present invention, as an effective material used for protecting nucleic acid and/or inhibiting cell apoptosis. Some materials useful for protecting nucleic acid and/or inhibiting cell apoptosis can be used to prevent, alleviate or treat degenerative diseases such as Alzheimer's disease (AD), Parkinson's Disease (PD), and the like.

Based on the new discovery of the present invention and the combination with current common knowledge, those skilled in the art can prepare or design agonists or inhibitors of AChE, and these agonists or inhibitors are also included in the present invention.

As a preferred embodiment of the present invention, said AChE agonists include (but not limited to): plasmids expressing acetylcholinesterase, agents which direct acetylcholinesterase or its encoding gene to enter cell nucleus (such as nuclear importer), magnesium ions, calcium ions or materials which can provide magnesium ion and/or calcium ion.

Said AChE agonist damages nucleic acid or promotes cell apoptosis by affecting AChE. Thus, it can be used for preparing a drug, particularly antitumor drug, to damage nucleic acid or promote cell apoptosis.

Said AChE antagonist can be a nucleic acid inhibitor, protein inhibitor, antibody, ligand, proteolytic enzyme, protein-binding molecule, EDTA, which can downregulate the expression of acetylcholinesterase or the encoding gene thereof. As a preferred embodiment of the present invention, said AChE antagonist is a nucleic acid inhibitor designed based on AChE gene sequence, such as a small interfering molecule. Preferably, said small interfering molecule has a sequence of SEQ ID NO:1 or SEQ ID NO:2. Designing interfering molecules according to specific target sequence is known to those skilled in the art, and plasmids currently used to construct interfering molecules are commercially available. Said interfering RNA molecule can be delivered into cell by suitable transfection reagents, or can be delivered into cell using various techniques known in the art. According to known proteins, screening of antibodies (monoclonal antibody or polyclonal antibody) whose activity can be inhibited is also a routine technique in the art.

Said AChE inhibitor exerts its protecting effect on nucleic acid or inhibiting cell apoptosis by affecting AChE. Especially when cell apoptosis occurs, AChE would enter nucleus, and treatment with AChE inhibitor at this time can prevent AChE from entering nucleus, or inhibit the expression or activity of AChE, in order to inhibit cell apoptosis. For some beneficial cells which can be led to apoptosis resulted from AChE entering to nucleus, they can be protected using AChE inhibitor, to slow down its apoptosis process or inhibit its apoptosis.

### Materials to be transported to nucleus

Nucleic acid is generally present in cell nucleus, therefore, some materials which may direct AChE or the agonist or antagonist thereof to enter nucleus are useful materials for regulating the stability of nucleic acid or regulating cell apoptosis. Thus, the present invention also provides a material including (1) AChE or its agonists or antagonist; and (2) a nuclear importer which directs AChE or the agonist or antagonist thereof to enter nucleus. (1) mutually combines with (2) by means of covalent linkage, coupling, molecular force action, charge absorption or charge attachment, encapsulation or embedment and other ways.

A nuclear importer may be any molecule directing protein or its encoding gene to enter nucleus, as long as it has a targeting effect on cell nucleus. For example, said nuclear importer may be (but not limited to): nuclear localization signal (NLS), karyopherin or the core fragments thereof (retaining nuclear localization or nuclear transport function), steroid hormone, virus, viral protein or virus-like particle, cationic polymer, radionuclide, nanosphere.

Guides for transport to nucleus include: Simian Virus 40 (SV40) large T NLS, M9 NLS, c-myc NLS, nucleoplasmin NLS, Xenopus N1 NLS, FGF3 NLS, and PARP NLS, antennapedia peptide, TAT peptide, c-myc peptide, VirD2 peptide, nucleoplasmin peptide, aryl hydrocarbon receptor nuclear translocator (ARNT) peptide, a polyoma large T antigen nuclear localization signal sequence, an adenovirus E1a nuclear localization signal sequence, and an adenovirus E1b nuclear localization signal sequence, the adenoviral NLS, the HIV-I Tat protein NLS, the lymphoid enhancer factor 1 NLS, an IBD-oligolysine peptide, a dual function CD46 targeting peptide/adenoviral NLS, H2B, v-Jun, Dorsal, NIN2, SWI5, RB, PTHrP, HnRNP, Pho4, rpL23a, rpL25, Protamine NLS, MTA1, CBP80, Rev, STAR NLS, hTAP, Mata2 NL.

Alternatively, virus, viral protein or virus-like particle are selected from (but not limited to): JC virus, JC virus VP1 protein, JC virus-like particle (VLP), chicken anemia virus, chicken anemia virus VP3, Apoptin.

Said nuclear importer may be tumor cell-specific, e.g., virus, viral protein or virus-like particle. More specifically, such as JC virus (JCV), which can form virus-like particle (VLP), and encapsulate fluorescent dye Cy3 or drug wherein and enter nuclear via transportation directly; or such as VP1 protein of JC virus (JCV), which can transport the target protein into nuclear, may be prepared into a fusion protein with AChE (see Chinese journal of microbiology and immunology, Vol 2, 2005, JC virus-like particle transports into nuclear). Additional nuclear importer can be, for example, viral protein 3 (VP3 or Apoptin) from Chicken Anemia Virus, wherein the C-terminal of the protein has tumor cell-specific nuclear localization signal (tNTS), which may be effectively located at the nucleus of a tumor cell or a metastatic tumor cell (See Drug Resist Updat. 2006 Feb-Apr;9(1-2):40-50. Epub 2006 Apr 18; Tumor-specific nuclear targeting: promises for anti-cancer therapy?). Additional nuclear importer can be a cationic polymer, e.g., β-carboxyl amidated cationic polymer such as polyethyleneimine (PEI) and Poly-L-Lysine (PLL), which are novel carriers for nucleus-targeting drugs reversing from negative charges to positive charges, for directly delivering drug to the nucleus of a cancer cell, which can encapsulate an antitumor drug such as AChE and efficiently deliver the drug to the nucleus (See Polymer Bulletin, Email of the Editorial Board, N0.12, 2010, Targeted Charge-Reversal Nanocarriers for Nuclear Drug Delivery).

Said nuclear importer may also be some tumor cell-nonspecific materials, such as Auger-emitting radionuclides, such as (99m)Tc, see J Biol Inorg Chem. 2011 Jun 25. [Epub ahead of print]; Nuclear targeting with cell-specific multifunctional tricarbonyl M(I) (M is Re, (99m)Tc) complexes: synthesis, characterization, and cell studies. Or such as persulfated molecular umbrella, see Bioconjug Chem. 2008 Aug;1(8):1510-3. Epub 2008 Aug 6.Cellular entry and nuclear targeting by a highly anionic molecular umbrella. Or such as glucose-derived carbon nanospheres, see Nano Lett. 2008 Oct;8(10):3182-8. Epub 2008 Sep 19.Intrinsically fluorescent carbon nanospheres as a nuclear targeting vector: delivery of membrane-impermeable molecule to modulate gene expression in vivo. Or such as estradiol-pheophorbide, see Photochem Photobiol Sci. 2006 Nov;5(11):996-9. Epub 2006 Oct 9.Synthesis of estradiol-pheophorbide a conjugates: evidence of nuclear targeting, DNA damage and improved photodynamic activity in human breast cancer and vascular endothelial cells.

As a preferred embodiment of the present invention, said AChE or the agonist or antagonist thereof (or its complex with nuclear importer) can also mutually combine with cell- or tissue-targeting materials by means of covalent linkage, coupling, molecular force action, charge absorption, attachment, encapsulation or embedment and other ways. Said cell- or tissue-targeting materials include: binding molecules which recognize surface molecules on specific cells (e.g., antibody (preferably monoclonal antibody), ligand, small chemical molecule or polypeptide), steroid hormones. For example, when said cell is a breast cancer cell, said binding molecule is an anti-Her2 antibody; when said cell is a prostate cancer cell, said binding molecule is a LHRH polypeptide.

As a preferred embodiment of the present invention, said AChE or the agonist or antagonist thereof (or its complex with nuclear importer) can also be mutually combined with protein transduction domain polypeptide by means of covalent linkage, coupling, molecular force action, charge absorption, attachment, encapsulation or embedment and other ways. Protein transduction domains which have already been found include TAT, Antp and VP22 etc. (Beerens A M J, Al Hadithy A F Y, Rots M G, et al. Protein transduction domain and their utility in gene therapy. Curr Gene Ther, 2003 ,3 (5) :486 ~ 494). Non-toxic peptide naturally derived from protamine is called low molecular weight protamine (LMWP). It has a relatively high Arg content, and important sequence similar to TAT (currently known as a very important protein transduction domain polypeptide). Therefore, LMWP is capable of penetrating biomembrane, making large protein molecules and the like otherwise can not penetrating biomembrane enter a cell and retain protein function. For example, a protein transduction domain polypeptide, as reported by China Biotechnology; May 2004, Vol 24, No.5, "TAT peptide and nuclear localization signal mediated protein delivery into cell nuclei". For example, a protein transduction domain polypeptide, as reported by Proc Natl Acad Sci U S A. 2002 Apr 2;99(7):4489-94. Epub 2002 Mar 19.Ability of the hydrophobic FGF and basic TAT peptides to promote cellular uptake of recombinant Cre recombinase: a tool for efficient genetic engineering of mammalian genomes, by a method of linking target protein (such as AChE) with protein transduction domains (PTDs), and then directly delivering target protein into cell through PTD action.

As a preferred embodiment of the present invention, said material useful for regulating nucleic acid stability or cell apoptosis is a fusion protein of AChE with a nuclear transport signal molecule; preferably, a fusion protein of AChE with a nuclear localization signal (NLS).

Said nuclear localization signal (NLS) of the present invention may be a nuclear localization signal containing core sequence (Lys-Arg/Lys-X-Arg/Lys), not having acidic amino acids and amino acids of macromolecules, see HallMN, Craik C, Hiraoka Y. Homeodomain of yeast rep ressor al2 pha 2 contains a nuclear localization signal. Proc Natl Acad Sci USA, 1990, 87: 695426958.

The preparation of fusion proteins is known to those skilled in the art, and between said AChE and nuclear transport signal molecule, a polypeptide linker (linker peptide) can be included or not. Preferably, said fusion protein is formed by fusing SEQ ID NO: 5 or aa 1-547 thereof with an amino acid sequence encoded by nucleotide sequence of SEQ ID NO: 3, between which some linker peptide sequences can be included. Preferably, said fusion protein has an amino acid sequence shown by SEQ ID NO: 6.

Nucleic acid encoding said fusion protein is also included in the present invention, the nucleic acid can be formed by linking nucleic acid sequences of natural AChE or NLS, or the variants or degenerating forms thereof. Carriers comprising said fusion protein-coding nucleic acid molecule are also included in the present invention. Said carrier also includes expression regulating sequence operably linked to the sequence of said nucleic acid molecule, for expressing said fusion protein. Host cells comprising said fusion protein-coding nucleic acid are also included in the present invention.

After the complexes of AChE of the invention with the nuclear importer, or the constructs encoding the complex of AChE with the nuclear importer are directed into a cell, they can unexpectedly promote apoptosis in cells (including tumor cells), which is very useful for eliminating harmful cells (such as tumor cells). Therefore, the complex of AChE with a nuclear importer is an excellent antitumor molecule.

The present invention demonstrated in Examples for the first time that after introducing *NLS-AChES* gene encoding the fusion protein of AChE and nuclear transport signal molecule into various tumor cells, significantly cell apoptosis were induced, and the apoptosis rate was about 100%; furthermore, *NLS-AChES* gene completely inhibited the subcutaneous tumor-forming capability of human tumor cells in nude mice. The experimental data demonstrated that, *NLS-AChES* gene was a novel tumor suppressor gene. For inducing cell apoptosis, or for the universality for cell types upon which it effects, *NLS-AChES* fusion gene has incomparable superiority. *NLS-AChES* fusion gene is a more effective "tumor killer" gene, a novel leader gene for antitumor gene therapy drug, expected to bring new prospects for the recovery of cancer patients.

Based on repetitive verification of above experiments, the inventors concluded that: *NLS-AChES* fusion gene can efficiently promote cell apoptosis after overexpressing in tumor cells; and can lead to highly efficient death of malignant cells in vivo through the targeting expression of this gene started by a vector comprising a tumor tissue-specific promoter. Some tumor tissue-specific promoters are, for example, shown in table 1.

**Table 1**

| **Promoter** | **Tumor Type** |
|---|---|
| carcino-embryonic antigen (CEA) promoter | human colorectal adenocarcinoma cell LS-174T; colon cancer cell SW480; lung adenocarcinoma cell A549; epithelial origin tumor cell |
| alpha fetoprotein (AFP) promoter | liver tumor cell |
| survivin(SUR) promoter | liver tumor cell; prostate cancer cell |
| human telomerase reverse transcriptase (hTERT) gene promoter | lung cancer, colon cancer, breast cancer, cervical cancer, liver tumor, leukemia |
| secretory leukoprotease inhibitor (SLPI) promoter | ovarian cancer; laryngeal cancer |
| squamous cell carcinoma antigen 2 (SCCA2) promoter | squamonus cell carcinoma of lung; laryngeal cancer |
| prostate cancer-specific promoter PSAe(AREc)-PSMAe-TARPp[P(A)PTp] | prostate cancer |
| prostate specific antigen (PSA) promoter | prostate cancer cell |
| tyrosine kinase gene promoter 5'-end specific promoter of tyrosinase protein 1 gene | melanoma cell |
| epithelial mucin (MUC)-1 gene promoter | breast cancer cell |
| tumor drug resistance gene promoter | |
| tissue-specific promoter OSP-1(ova-specificpromoter-1) | ovarian cancer |
| PSMA promoter | prostate cancer |
| multidurg resistance gene (mdrl germ) promoter | |
| breast cancer-specific gene 1 (*BCSG1,* also called *Synuclein γ*) promoter | breast cancer |

*AChES* has the following advantages: first, the introduction of single gene can effectively induce tumor cell apoptosis; second, location in the downstream of the apoptosis signalling pathway, such that the function of the introduced wide-type gene is not interfered by other gene mutations in the apoptosis pathway. In addition to the introduction of single *AChES* gene resulting in a tumor cell apoptosis rate of approximately 100%, the gene's location in the most downstream of the AChE apoptosis signalling pathway, also allows its protein exerts DNA degradation function directly after transport to the nucleus, and the wide-type *AChES,* after introduced to tumor cells, can thus function without restricting by other gene mutations in the pathway.

### Vectors and cells

The present invention also includes the vector (expression vector) comprising the nucleic acid encoding AChE protein (or the bioactive fragments thereof), or the nucleic acid encoding AChE protein (or the bioactive fragments thereof) and nuclear importer and/or cell- or tissue-targeting material and/or protein transduction domain polypeptide. Said expression vector can additionally comprise expression regulatory sequence operably linked to said nucleic acid sequence for protein expression. The term "vector" refers to a bacterial plasmid, phage, yeast plasmid, virus such as plant cell virus, mammal cell virus (e.g., adenovirus, retrovirus) or other vectors well known in the art; including prokaryotic vector and eukaryotic vector. In a word, any plasmid and vector can be used as long as they can replicate and remain stable in the host. Typically, an important characteristic of expression vectors is containing origin of replication, promoter, marker genes and translation-controlling elements.

Methods well known to those skilled in the art can be used to construct expression vectors containing nucleic acid encoding said AChE protein (or its bioactive fragments) and nuclear importer and/or cell or tissue-targeting materials. These methods which are well known by one skilled in the art include in vitro recombinant DNA techniques, DNA synthesis techniques, in vivo recombinant techniques, etc. Said DNA sequence can be effectively connected to a suitable promoter in an expression vector in order to guide mRNA synthesis. Representative examples of these promoters are: E.coli lac or trp promoter; λ phage P_{L} promoter; eukaryotic promoter, including CMV immediate early promoter, HSV TK promoter, SV40 early and late promoters, LTRs of retrovirus and some other promoters known to control gene expression in prokaryotic or eukaryotic cells or in virus. Expression vector also includes ribosome bind site for translation initiation and transcription terminator. An expression vector preferably comprises one or more selective marker genes to provide phenotypic traits for selecting transformed host cells, such as dihydrofolate reductase, neomycin resistance and green fluorescent protein (GFP) for culturing eukaryotic cells, or tetracycline or ampicillin resistance for E.coli.

Vectors comprising appropriate DNA sequences described above and appropriate promoters or controlling sequences can be used to transform appropriate host cells, to allow expression of the proteins. Or they can be administered to subject in need of treatment directly as gene therapy materials.

When the polynucleotides of the invention are expressed in higher eukaryotic cells, if an enhancer sequence is inserted in the vector, the transcription would be improved. An enhancer is a cis-acting factor of DNA, usually about 10-300 base pairs, acting on the promoter to enhance gene transcription.

In addition, recombinant cells containing nucleic acid encoding said AChE protein (or its bioactive fragments) and nuclear importer and/or cell or tissue-targeting materials are also included in the present invention. "Host cells" include prokaryotic cells and eukaryotic cells. Common prokaryotic host cells include E.coli, Bacillus subtilis etc.; e.g., Escherichia coli cell (E.coli), such as E.coli HMS174(DE3), or BL21(DE3). Common eukaryotic host cells include yeast cell, insect cell and mammal cell.

### Composition

The present invention also provides a composition for regulating DNA damage or cell apoptosis, which contains an effective amount (e.g., 0.000001-50 wt%; preferably 0.00001-20wt%; more preferably, 0.0001-10wt%) of above-mentioned regulatory substances (including: substances consisting of said AChE protein or the agonist or antagonist thereof, or acetylcholinesterase or the agonist or antagonist thereof, and a nuclear importer and/or cell- or tissue-targeting material and/or protein transduction domain polypeptide), as well as a pharmaceutically acceptable carrier.

Generally, these substances may be formulated into a non-toxic, inert and pharmaceutically acceptable aqueous vector medium, wherein the pH is usually about 5-8, preferably, pH is about 6-8.

As used herein, the term "effective amount" or "effective dose" refers to the amount which can exert function or activity on human and/or animal and is acceptable for human and/or animal.

As used herein, "pharmaceutically acceptable" ingredients are materials applicable to human and/or mammal without excessive adverse side effects (e.g., toxicity, irritation and allergic reaction), i.e., with reasonable benefit/risk ratio. The term "pharmaceutically acceptable carrier" refers to carriers for administrating therapeutic agents, including various excipients and diluents.

The composition of the invention comprises a safe and effective amount of above-mentioned regulatory substances as well as a pharmaceutically acceptable carrier. These carriers include (but not limited to): saline, buffer, glucose, water, glycerol, ethanol, and combinations thereof. Pharmaceutical formulations usually should match administration routes, and the composition of the present invention can be prepared into injection forms, for example, prepared using physiological saline or aqueous solution containing glucose and other adjuvants by routine methods. Said composition is advantageously produced under aseptic conditions. The administration amount of the active ingredients is the therapeutically effective amount. The composition of the present invention may also be produced as a controlled- release formulation.

The effective amount of the regulatory substances described by the present invention can vary according to mode of administration and severity of diseases to be treated. Selecting preferred effective amount can be determined by an ordinary skilled in the art according to various factors (e.g., by clinic trials). Said factors include but not limited to: pharmacokinetic parameters of said AChE protein or the agonist or antagonist thereof, such as bioavailability, metabolism, half-life etc.; the severity of the disease to be treated in a patient, weight and immune state of patient, administration route etc. Normally, when AChE protein or the agonist or antagonist thereof of the present invention is administered by the dose of approximately 0.00001mg-50 mg/kg animal weight (preferably, 0.0001mg-10mg/kg animal weight), satisfactory effect could be obtained. For example, due to the urgent demand of therapeutic status, separate doses may be administered for several times daily, or the doses may be reduced proportionally.

When said regulatory substances are substances consisting of AChE protein or the agonist thereof, or acetylcholinesterase or the agonist thereof, and a nuclear importer, said composition can be prepared as an antitumor drug, which has been proven to have an excellent antitumor effect.

### Drug screening

After knowing new functions of said AChE protein, we can screen materials regulating AChE transcription, expression, activity or nucleus importing based on this feature, in order to find drugs which can affect nucleic acid stability or cell apoptosis by influencing AChE.

Therefore, the present invention provides a method for screening potential materials which can regulate cell apoptosis or nucleic acid stability, wherein the method comprises: treating a system expressing or containing AChE with candidate materials; and detecting the transcription, expression or activity of AChE in said system; wherein, if said candidate materials can improve the transcription, expression or activity of AChE, it indicates that the candidate materials are potential materials to damage nucleic acid or promote cell apoptosis; if said candidate materials can reduce the transcription, expression or activity of AChE, it indicates that the candidate materials are potential materials to protect nucleic acid or inhibit cell apoptosis.

In a preferred embodiment of the present invention, said system expressing or containing AChE is a cell system, which thus further includes: observing the nucleus importing of acetylcholinesterase or its encoding gene, if the proportion of acetylcholinesterase or its encoding gene in cell nucleus increases, it suggests that the candidate materials are potential materials to damage nucleic acid or promote cell apoptosis; if the proportion of acetylcholinesterase or its encoding gene in cell nucleus decreases, it suggests that the candidate materials are potential materials to protect nucleic acid or inhibit cell apoptosis.

Under the suggestion of the present invention, all those skilled in the art can preliminarily select some candidate materials, such as materials considered as potentially useful materials. For example, said candidate materials include (but not limited to) interfering molecule, nucleic acid inhibitor, binding molecule (e.g., antibody or ligand), small molecule compound designed for acetylcholinesterase or its encoding gene; or recombinant plasmid expressing acetylcholinesterase; or fusion protein of acetylcholinesterase and a nuclear importer, or its encoding gene, etc.

When screening, to observe the changes of AChE expression or activity more easily, a control group may be set up, wherein said control group may be a system expressing AChE without adding said candidate materials.

Alternatively, based on the new discovery of the present invention, an anti-AChE nuclease activty drug can be screened, e.g., anti-AD and anti-PD drug. Method used in the past for screening ACHE inhibitor is through screening drug to treat AD using esterase activity of ACHE, because drugs which can inhibit esterase activity of ACHE are ACHE inhibitors. However, drugs which inhibit esterase activity of ACHE may not inhibit cell apoptosis, even may not inhibit DNase activity. The inventors found that ACHE had nuclease activity, and new nuclease activity was unrelated with esterase activity or functional domain. Therefore, the screening of nuclease inhibitor of ACHE should be based on the determination of whether the enzyme is capable of cleaving DNA. The new method of the present invention is a method which can be used to determine whether certain protein is capable of cleaving DNA. Afterwards, when screening drugs to treat AD, only minimal ACHE fragment is needed, e.g., a fragment at position 2-138 of SEQ ID NO:5, or smaller one such as fragment of aa 33-138, which has the effect of cleaving DNA; if nuclease activity would be lost after adding candidate inhibitor, it indicates that the candidate inhibitor is effective of anti-apoptosis as well as anti-AD.

As a preferred embodiment of the present invention, said method further includes: performing further cell experiments and/or animal tests on the obtained potential materials, to further select and determine materials useful for regulating cell apoptosis or nucleic acid stability.

As a preferred embodiment of the present invention, when screening using AChE, preferred is AChE fragment not including all or parts of AChE esterase activity key sites (e.g., position 234 or the adjacent position thereof, position 365 or the adjacent position thereof and/or position 478 or the adjacent position thereof in acetylcholinesterase of human origin), so that the obtained materials through screening function by regulating AChE nuclease activity sites.

On the other hand, the present invention provides potential materials regulating cell apoptosis or nucleic acid stability which can be obtained by said screening method. These preliminarily screened materials may constitute a screening library.

Major advantages of the present invention are:
(1) The present invention discloses for the first time a new function of AChE, i.e., the function of nuclease, and provides a new understanding on the physiological process of DNA degradation in apoptotic cells.
(2) The present invention discloses for the first time that, the importation of the combination of AChE and a nuclear importer into nucleus are very efficient in promoting cell apoptosis, which may be used as a novel "killer" of tumor. Based on the present invention, novel antitumor gene therapy drugs can be developed to overcome current therapeutic dilemma of malignant tumors and bring new hopes for cancer patients.

The present invention is next described in detail in conjunction with specific embodiments\. It should be understood that these embodiments are used merely to illustrate the present invention and are not used to limit the scope of the present invention. The specific conditions of experimental methods are not indicated in the following examples, usually in accordance with conventional conditions described by J. Sambrook et al, Molecular Cloning A Laboratory Manual, Science Press, 2002, or in accordance with the conditions recommended by the manufacturer. Unless otherwise indicated, percentages and parts are calculated by weight.

Unless otherwise defined, all professional and scientific terms used herein have the same meaning as those familiar to person skilled in the art. In addition, any method and material similar or equal with the records can be used in the present invention. The preferred embodiments and materials described herein are only for demonstration purpose.

### 1. Materials and methods

### Cell culture

Human cervical cancer cell HeLa, colonic adenocarcinoma cell HT-29, glioma cell SK-N-SH, microvascular endothelial cell HMEC-1, embryonic kidney cell HEK 293T, rat pheochromocytoma cell PC-12, human colonic adenocarcinoma SW620 cell, liver cancer BEL-7404 cell were from American Type Culture Collection (ATCC).

MCDB131, DMEM, Opti-MEMI medium, horse serum were purchased from GIBCO-BRL; new-born calf serum was purchased from Hangzhou Evergreen Biological Engineering Materials Co., Ltd. HMEC-1 cells were cultured in complete MCDB131 medium according to the manufacturer's recommendations (Invitrogen). When grew to a saturated state, the cells were transferred to a new gelatin-coated culture flask; cells were stable after 7 passages and then used for experiments. The storage culture and cell culture of PC-12 were in RPMI1640 medium (containing 10% FBS), respectively. HeLa, SK-N-SH, HT-29 and HEK 293T cells were in DMEM medium (containing 10% FBS).

### Reagents and materials

Rabbit anti-cleaved Caspase-3 polyclonal antibody (no. 9661), rabbit anti-PARP polyclonal antibody (no. 9542) were purchased from Cell Signaling technology, MA, USA; mouse anti-β-actin antibody (AC-15) was purchased from Sigma; goat anti-human AChE antibody (N-19), goat anti-Histone H3 antibody (C-16), mouse anti-α-Tubulin antibody (B-5-1-2), horseradish peroxidase conjugated goat anti-rabbit IgG second antibody were purchased from Santa Cruz Biotechnology; reverse transcriptase M-MLV was purchased from Promega Life Science; high fidelity enzyme KOD-Plus was purchased from TOBOYO; BCA was purchased from Pierce Biotechnology. TUNEL assay kit was purchased from Roche. Transfection reagent Lipofectamine 2000 was purchased from Invitrogen. G-actin protein was purchased from Cytoskeleton (Denver, CO, USA).

### Plasmids and siRNA

Plasmid containing sequence encoding S form of acetylcholinesterase (S-AChE protein) was obtained from The Hebrew University of Jerusalem, Israel. If desired, full-length AChE cDNA sequence of human origin (GenBank accession number: M55040) can also be cloned to mammal expression vector pEGFP-N1 (Clontech, Mountain View, CA), obtaining plasmid pEGFP-AChE expressing AChE-GFP. Two groups of siRNA oligonucleotide sequences directed at human AChE were as follows:
siRNA1, AAGUCUCCCGCGUUGAUGAGGGCCU (SEQ ID NO: 1);
siRNA2, AAGAAGCGGCCAUCGUACACGUCCA (SEQ ID NO: 2).

Sequence of nuclear localization signal SV40 NLS (containing HindIII enzyme cutting site AAGCTT at the end) was as follows: 5'-ATGGCTCCAAAGAAGAAGCGTAAGGTAGGACCAAAGAAGAAGCGTAAGGT T(SEQ ID NO: 3) AAGCTT-3'. (what were encoded was MAPKKKRKVG PKKKRKV; wherein core fragment was PKKKRKVG).

### Preparation of plasmid DNA

The small-scale preparation of plasmid: a single colony was picked and inoculated into 3ml LB culture solution, shook and cultured at 37°C for 12-16h, which is then centrifuged at 4000 rpm for 5 min to collect bacteria, and pipetted to dryness. Suspended and blended using pre-cooled 100 µl solution I (50 mM glucose, 25 mM Tris-HCl pH 8.0, 10 mM EDTA). 200 µl freshly-formulated Solution II (0.2 M NaOH, 1% SDS) 20 mL was added, and blended. Additional 150 µl pre-cooled Solution III (3 M NaAC, 5 M HAC, pH 4.8) was added and subjected to ice-bath for 3-5 min after blending. Centrifuged at 12000 rpm for 10 min. The supernatant was collected and equal volumes of phenol: chloroform: isopentanol were added and shook. Centrifuged at 12000 rpm for 5min at room temperature. Transferred supernatant, added 2x volume of ethanol to precipitate DNA; finally, rinsed and precipitated with 70% ethanol, subjected to vacuum drying, dissolved in an appropriate amount of TE (10 mM Tris-HCl, 1 mM EDTA, pH 8.0). For large-scale preparation of the plasmid, Plasmid Midi Kit (QIAGEN) can be used.

### Construction of recombinant plasmid

pEGFP vector was purchased from Clontech.

Construction of the plasmid expressing AChE-GFP: full-length *AChE* cDNA sequence of human origin (GenBank accession number: M55040) was cloned to BglII/EcoR sites of mammal expression vector pEGFP-N1 (purchased from Clontech), to obtain pEGFP-*AChE*.

Construction of the plasmid expressing AChE (1-547AA): 1-547AA coding sequence in AChE cDNA of human origin was cloned to BglII/EcoRI sites of expression vector pEGFP-N1, obtaining pEGFP-AChE (1-547AA).

Construction of the plasmid expressing AChE (1-548AA): 1-548AA coding sequence in AChE cDNA of mice origin (GenBank accession number: NM_009599.3) was cloned to BglII/EcoRI sites of expression vector pEGFP-N1, to obtain pEGFP-*AChE* (1-548AA).

Construction of the plasmid expressing Histone H2b-RFP fusion protein: pDsRed2-N1 was purchased from Clontech; conventional Histone H2B gene of human origin was cloned to the space between SalI/NotI restriction enzyme cutting sites of above-mentioned plasmid.

Construction of the plasmid expressing Tubulin-GFP fusion protein: pEGFP-c1 plasmid (purchased from Clontech); conventional tubulin gene of human origin was cloned to the space between Bgl II/Sal I restriction enzyme cutting sites of above-mentioned plasmid.

Construction of the AChE plasmid of human origin with nuclear localization signal (NLS): obtained cDNA sequence encoding AChE of human origin, added DNA encoding NLS sequence (MAPKKKRKVGPKKKRKV) before its 5'-end (without ATG), obtained fusion gene, inserted into BglII/EcoRI sites of pEGFP, to obtain pEGFP-*NLS*-*AChE*.

Based on pEGFP-*NLS*-*AChE*, mutation of the *AChE* coding sequence thereof (corresponding to position 234, 365, 478 of human *AChE,* i.e., S234A, E365A, H478A mutation) was performed using conventional site-directed mutagenesis.

### Deletion mutants of AChE protein molecule

To investigate the specific site functioning as nuclease in AChE protein, a series of mutants were constructed, including: position 2-398, position 2-247, position 2-191, position 2-138, position 2-72 (note: the calculation of sequence site was based on AChE protein sequence from human origin of SEQ ID NO:5).

After obtaining DNA sequence of above-mentioned protein fragment, added NLS sequence in its 5'-end. First, NLS sequence was inserted into BglII/EcoRI sites of expression vector pEGFP, to obtain pEGFP-*NLS* vector. Then, between HindIII/EcoR sites of the vector, inserted 4bp-1194bp, 4bp-741bp, 4bp-573bp, 4bp-414bp, 4bp-216bp sequences of wild type AChE sequence (SEQ ID NO: 4), i.e., acquired *pEGFP-NLS-AChE* (2-398AA), *pEGFP-NLS-AChE* (2-247AA), *pEGFP-NLS-AChE* (2-191AA), *pEGFP-NLS-AChE* (2-138AA), *pEGFP-NLS-AChE* (2-72AA), respectively.

### Cell treatment and conventional plasmid transfection

In general, cells were put in DMEM culture solution contaning 10% new-born calf serum, streptomycin (100 µg/ml) and penicillin (100 units/ml), cultured in 5% CO₂ incubator, 37°C. Cells were counted and passaged at 5×10⁴ cells/ml. Before treatment, cells were inoculated into 6-well plate with certain density, cultured to reach about 70% confluency after 18h. Blended 2 µg plasmid and 125 µl Opti-MEMI culture solution with serum but no antibiotics (solution A), placed at room temperature for 5 min. Blended 5µl Lipofectamine 2000 and 125 µl Opti-MEMI culture solution with serum but no antibiotics (solution B). Combined solution A and solution B, placed at room temperature for 20 min after blending. Pipetted antibiotics-containing culture solution from culture plate, added 750µl Opti-MEMI culture solution with serum but no antibiotics to each well to cover cells, and added 250 µl above-mentioned Lipofectamine-DNA mixture, and gently shook and blended. Cultured for 6h under 37°C, 5% CO₂. Pipetted away transfection mixture, re-added 1500 µl DMEM complete culture solution, continued to culture.

### Transient transfection plasmid for tumor cells

Transfection of cells implanted in Φ10cm petri dish: tumor cell seeding density: 2.8×10⁶/dish. After implanted for 24h, cells were divided into 3 groups:
Control group I: transiently transfected with pEGFP vector;
Control group II: transiently transfected with pEGFP- *AChES*;

Experimental group: transiently transfected with *pEGFP-NLS-AChES*.

Transfection mixture was formulated as follows: OPTIMEM 750 uL/dish, FuGENE HID reagent (Roche) 45ul/dish, total amount of plasmid was 15ug/dish. After gently shaking and standing at room temperature for 10 min, finally dropwise added to cell dish.

Transfection of cells implanted in 24-well plate: cell seeding density 8×10⁴/dish; in transfection mixture, OPTIMEM 25 uL/well was contained; FuGENE HD reagent (Roche) 1.5ul/well, total amount of plasmid was 500ng/well.

The remaining parameters were the same as above.

### Conventional flow cytometry sorting or analysis

Cells were washed once with pre-cooled PBS after trypsinization, centrifuged to collect, and then fixed overnight in -20□ by using 70% ethanol. Fixed cells were centrifuged to remove ethanol, washed twice with pre-cooled PBS. Cells were suspended in PBS containing 200 µg/ml RNase A and 50 µg/ml PI (propidium iodide), placed in 37°C for 30 min, accomplished flow cytometry analysis by FACS calibur from Becton & Dickinson; result analysis was performed by FCS3.0 software, with at least 10⁶ cells each time. For flow cytometry analysis of GFP positive cells, cells were washed once with pre-cooled PBS after trypsinization, centrifuged to collect, fixed for 10 min with 4% paraformaldehyde, washed twice with pre-cooled PBS. Permeabilized for 5 min at room temperature using 0.1%(v/v) Triton x-100/0.1%(w/v) sodium citrate solution, and then washed twice by pre-cooled PBS. Cells were suspended in PBS containing 200 µg/ml RNase A and 50 µg/ml ethidium iodide and placed in 37°C for 30 min, and then accomplished flow cytometry analysis by FACS calibur from Becton & Dickinson; result analysis was performed by FCS3.0 software, with at least 10⁶ GFP positive cells each time. Cells which need sorting were suspended by 37°C-preheated culture solution after trypsinization, and directly subjected to BD FACS Aria II sorting.

### Flow cytometer screening of tumor cell

After cells were transfected by relevant plasmids for 24h, cells were collected by trypsinization method, suspended in culture solution after centrifugation (300uL/Φ10cm dish). The working concentration of penicillin in the medium was 100U/mL, and the working concentration of streptomycin was 100ug/ml. After that, green fluorescent protein strong positive region cells distant from negative cell region were obtained by computer sorting. Finally, cells after sorting were implanted in 96-well plate, with 2×10⁴/well, implanting 12 parallel wells per group.

### Cell MTT assay

After flow cytometry sorting, cells were subject to MTT assay at different timepoints (e.g., 0, 8, 24, 48, 72h), respectively, to determine absorbance value under 570nm for cell growth curve. Specific operation was as follows: at 4h before detection, 20ul MTT stock solution (5mg/ml) (purchased from Sangon Biotech) was directly added to each well in all groups, placed in incubator to culture for further 4h; discarded supernatants from all wells, added DMSO 100µL, respectively, shook at room temperature for 10 min; solvent DMSO was used for zero adjustment, OD values of each sample under 570nm were measured with a microplate reader.

### TUNEL method for detecting cell apoptosis

At 24^{th}h after cells were transfected with relevant plasmids, cell culture solution was discarded, and the cells were washed once with PBS at room temperature for 5 min. Fixd with 4% paraformaldehyde at room temperature for 10 min; washed three times with PBS, 5 min/time. 0.5%Triton-X100 for permeabilizing membrane, at room temperature for 10 min. Washed three times with PBS, 5 min/time. 50µl TUNEL reaction mixture (Roche) reacted for 1h in 37°C. Washed three times with PBS, 5 min/time. 10µg/mL Hoechst33258 250uL was added to stain nucleus, staining at room temperature for 1 min, washed three times with PBS, 5 min/time. Observed under confocal laser scanning microscope and took photos.

### Determination of AChE esterase activity

Relative AChE enzyme activity in cell extract was determined by spectrophotometer in 405 nm using 96-well plate according the method of Ellman et al.(Ellman GL, Courtney KD, Andres V, Jr., Feather-Stone RM. A new and rapid colorimetric determination of acetylcholinesterase activity. Biochem Pharmacol 1961; 7: 88-95). Collected cells were washed twice by PBS, suspended in extract (50mM potassium phosphate buffer pH7.4, 1M NaCl, 0.5%Tween-20) after centrifugation, placed on ice for 1h; then centrifuged at 12000rpm for 10 min to take protein supernatant; total protein amount was determined by BCA method. According to the determined total protein amount, equal amount of total protein was taken from each sample to determine relative AChE activity. An unique inhibitor of butyrylcholine esterase (BChE), iso-OMPA (final concentration is 0.75 mM) was added to a system measuring activity in advance for 30 min, in order to eliminate possible interference from butyrylcholine esterase.

One active unit (U) means AChE hydrolyzed 1µmol substrate per minute in 37°C. When indicating relative AChE activity, AChE activity for transfecting empty vector cell lysate was set to 1, and the activities of other processed samples were expressed as its multiples. When determining activity of purified AChE protein, measured value from 1 µg BSA protein was set to 1, and the activities of other processed samples were expressed as its multiples.

### Western blotting

Cells treated or not treated by drug were centrifuged to collect after trypsinization, dissolved in 1×SDS PAGE gel loading buffer (50 mM Tris pH 6.8, 100 mM DTT, 2% (w/v) SDS, 10% (v/v) glycerol, freshly adding 1 µg/ml aprotinin and 10 mM PMSF) and boiled for 10 min. Protein concentration was determined by BCA method. Equal amounts of samples were loaded after adding bromophenol blue for SDS PAGE gel electrophoresis, transferred to membrane, blocked in 5% skim milk powder-containing TBST (10 mM Tris-HCl pH 7.8, 100 mM NaCl, 0.05% Tween-20) for 1h, and then, corresponding primary antibodies diluted with 5% skim milk powder TBST: anti-AChE antibody diluted at 1:500, anti-PARP antibody diluted at 1:1000, anti-Histone H3 antibody diluted at 1:3000, anti- Tubulin antibody diluted at 1:2000, anti-α-actin antibody diluted at 1:4000, were added, and incubated in 4°C for 12h. After washing by TBST for 3 times, corresponding HRP conjugated anti-goat, anti-rabbit or anti-mouse IgG secondary antibodies (diluted by 5% skim milk powder), were added, and incubated in 37°C for 1.5h. Finally, development was performed using ECL method and the results were detected by LAS-4000 of FUJIFILM.

Subcellular localization of AChE was observed by fluorescence microscope, confocal fluorescence microscopy, or intravital microscopy. Cells were cultured on coverslips, and drug or plasmid transfection was performed. The cells were fixed in 4 °C with 1 ml 4% paraformaldehyde for 40 min. For detecting endogenous proteins, 800 µl permeabilizing solution (0.1% Triton X-100 and 0.1% sodium citrate dissolved in PBS) was used at 37 °C to permeabilize for 5-10 min, and then washed three times with PBS. Blocking using 100 µl blocking solution (3% BSA dissolved in PBS) for 40 min. Dilution was performed with an appropriate ratio using anti-AChE antibody, before incubation in 4 °C overnight. Washing for three times with PBS, and incubating at room temperature with 80 µl secondary antibody (1/100 diluted in blocking solution) for 2h before washing three times with PBS. After PBS washing, staining with Hoechst 33258 staining solution in the dark for 10 min. Mounting with 50% glycerol. Subcellular localization of AChE was observed by Olympus IX 51 fluorescence microscope, or Leica TCS SP5 laser confocal fluorescence microscopy. For Live cell imaging, cells were directly placed on Leica Multi-Dimensional Workstation for Live Cell Imaging for observation.

### Purification of AChE protein

AChE protein used in *in vitro* system was purified as follows: transfecting HEK 293 cells and screening the cell strain stably expressing polypeptide with amino acid sequence of AChE1-547AA (Human) or AChE1-548AA (Mice) (retaining AChE activity, but removing the glycosylation site on Gly at position 557 and the hydrophobic C-terminal, resulting in a soluble monomeric AChE protein), and culturing the cells in Ultraculture cell culture medium (Biowhittaker). Having secretory signal, AChE was secreted into the cell culture medium, and the culture medium was collected every three days. Purification was performed by affinity chromatography conjugated with a reversible AChE inhibitor, Fasciculins (Marchot P, Ravelli RB, Raves ML, Bourne Y, Vellom DC, Kanter J et al. Soluble monomeric acetylcholinesterase from mouse: expression, purification, and crystallization in complex with fasciculin. Protein Sci 1996; 5(4): 672-9). The resultant protein was further subjected to electrophoresis and the purity and activity of which was identified through detecting AChE esterase activity.

### Detection of in vitro DNase activity of AChE

Purified plasmid DNA was used as substrate for detection. In the experiment of cleaving of plasmid DNA, 0.15µg of plasmid DNA with a transfection-grade purity (pEGFP-C1 plasmid, Clotech) was added to 10 µl buffer system containing (5.0 mM Tris-HCl, PH 7.5; 2.5 mM CaCl₂; 5.0 mM MgCl₂). 1 µg (or an amount indicated in Examples) of purified AChE proteins of human or mouse origin (AChE 1-547AA corresponding to human; AChE1-548AA corresponding to mouse) was further added, and incubated in 37°C. Sampling at 6h (or timepoints indicated in Examples).

In the experiment for inhibitor of AChE esterase activity AChE protein and the inhibitor were present in the in vitro buffer system, and incubated in 37°C for 30 min, before further adding of substrate DNA and incubation for another 6h.

In the experiment for AChE elimination, protein G glass beads conjugated with AChE antibody or IgG were present in the in vitro buffer system, and incubated in 37°C for 30 min, and then centrifuged at 1000 rpm for 2 min; the supernatant was carefully transferred to another test tube (not to involve the precipitate as far as possible), and substrate DNA was further added to incubate for another 6h. Finally, an appropriate amount of DNA electrophoresis loading buffer in 1% agarose gel was added and electrophoresis was carried out for 30 min in 1×TAE buffer, before taking photos under ultraviolet light.

### Immunohistochemistry assay of paraffin section

First, dewaxing and hydrating. Tissue chips were roasted in 60°C incubator for 30 min, soaked in xylene I, xylene II, xylene III for 10 min, respectively; soaked in mixture of xylene and alcohol (V:V=1:1) for 5 min; soaked in absolute ethyl alcohol I, absolute ethyl alcohol II, 95%(v/v) alcohol, 85% alcohol, 70% alcohol for 5 min, respectively; soaked in 50% alcohol for 2 min; soaked in distilled water twice, 3 min for each time. Next, pH6.0 citrate buffer (800-1500 mL) was used for antigen retrieval by high-pressure process. After retrieval, the temperature of the pressure cooker was reduced to room temperature. Slides were took out, washed twice with distilled water, 3 min for each time; washed twice with PBS (pH 7.2-7.4), 3 min for each time. 3%H₂O₂-Methanol mixture (30% H₂O₂: CH₃OH=1: 9) was used to block endogenous peroxidase at room temperature for 15 min. Washing three times with PBS, 5 min for each time. 0.5% TritonX-100 was used to permeabilize membrane for 15 min. Washing three times with PBS, 5 min for each time. Normal goat serum blocking solution was added dropwise at room temperature (20-28□) for 20 min. Shaking off the excess liquid, and primary antibody (N-19, purchased from Santa Cruz) was dropwise added directly and incubated in a wet box at 4□ overnight (≥18 h). Washing three times with PBS, 10 min for each time. The secondary antibody (A5420, purchased from Sigma) was added dropwise, 37°C for 30 min. Washing three times with PBS, 10 min for each time. Development was performed by DAB for 5-10 min, with the degree of staining under the microscope (in the dark) controlled. Just soaking in and washing with distilled water to stop staining. Haemotoxylin staining for 3-5 min, with nucleus showing violet blue; differentiation was made directly by 1% hydrochloric acid alcohol for 3-5 s, washing with tap water for 30 min before back to blue. Dehydration with85% alcohol, 90%, 95% alcohol I, 95% alcohol II, absolute ethyl alcohol II dehydration successively, and then dehydration with xylene I, xylene II, xylene III for 2 min, respectively. Finally, neutral balsam was used for mounting.

### Subcutaneous tumorigenicity assay of human BEL-7404 cells in nude mice

Cells were divided in two groups:
Control group: transfected with pEGFP plasmid;
Experimental group: transfected with pEGFP-*NLS-AChE.*

Transfection positive cells were sorted by flow cytometry, and centrifuged, resuspended in DMEM medium free of calf serum, at a cell density of 5×10⁶/100uL. The fluorescence of implanted cells was observed by stereo fluorescence microscope at 24hr, 72hr after implanting, and photographs were taken; from day 7 up to day 28, lengths and widths of tumors were measured using electronic caliper (Shanghai Shenhan Measuaing Tools co.,ltd) every three days. The tumor volumes were calculated and the tumor growth curve was plotted. On day 28, the nude mice were sacrificed by vertebrae breaking method, and photographs were taken to observe tumor formation.

### Statistical analysis

All the tests were repeated for at least three times. Results were expressed as mean ±SD. Analysis was performed by 2-tailed Student's t-test, and P < 0.05 was considered as significant difference.

### II. Examples

### Example 1. AChE expression increased in different cells induced by different methods, and AChE siRNA can prevent cells from apoptosis.

To verify that AChE expression increase is a conserved and universal phenomenon, four different cell strains of human origin (including Human cervical cancer cell HeLa, colonic adenocarcinoma cell HT-29, glioma cell SK-N-SH, microvascular endothelial cell HMEC-1) were treated by A23187 (purchased from Sigma) or Etoposide to induce apoptosis. In all the four cell strains, expression quantity of AChE was detected to be increased with the apoptosis processing (with PARP (poly(ADP-ribose)polymerase) cleavage as an apoptosis marker). See Figure 1A.

To further disclose the relationship between AChE expression and cell apoptosis, the inventors applied immunofluorescence staining on cells treated with hydrogen peroxide (50 µM) to induce apoptosis and found by obvservation that compared with normal cell group, apparent pyknosis occurred in nucleus of cells of apoptosis group, and AChE expression was up-regulated and AChE protein was co-localized with activated Caspase-3. See Figure 1B. The data show that AChE was involved in cell apoptosis induced by hydrogen peroxide.

To determine the role of AChE in cell apoptosis, the inventors designed two AChE siRNA (25mer), i.e., siRNA1 and siRNA2. These two groups of AChE siRNA both can effectively inhibit the increase in expression quantity of AChE in apoptosis, see Figure 1C.

HeLa cells were transiently transfected with siRNA1 and siRNA, after which HeLa cells transiently transfected with siRNA1 and siRNA were treated with Etoposide (50 µg/ml) to induce apoptosis, and then apoptosis rate was determined using flow cytometry (FACS). Compared with control group transfected with Scramble siRNA, DNA fragmentation degree was significantly reduced in cells transfected with AChE siRNA, and cell apoptosis was inhibited. But when transfected with AChE expression plasmid (plasmid containing S-AChE protein coding sequence) at the same time, the above-mentioned inhibition of apoptosis disappeared, see Figure 1D-E.

In sum, on the one hand, when inducing apoptosis by different methods in different types of cells, AChE expression were all up-regulated, and there was a positive correlation between its expression quantity and apoptosis process; on the other hand, AChE siRNA effectively protected cell from apoptosis. The above results indicated that, AChE was involved in cell apoptosis and played an important role in it.

### Example 2. AChE migrated to cell nucleus during apoptosis

To make sure which role does AChE play during cell apoptosis, first need to know subcellular localization of AChE before and after apoptosis. For this, confocal microscopy was used to observe whether the distribution of endogenous AChE before and after apoptosis has changed. The inventors chose 2 model cell strains: HeLa cell strains with no background expression of AChE and PC12 cell strains with relatively high background expression of AChE. The results showed that compared with normal growing cells, AChE wasn't only limited to cytoplasm region in apoptotic cells; importantly, its distribution changed, also presented nucleus distribution, see Figure 2A.

To further verify this result, the inventors performed cytoplasm-nucleus separation on PC12 and HeLa under normal growth and apoptosis, and employed immunoblot (IB) to detect AChE protein expression in cell nucleus and cytoplasm, respectively. AChE expression can't be detected in normal cultured HeLa; in PC12 cells, AChE expression was detected only in cytoplasm components, while couldn't be detected in nucleus. Different from above normal groups, AChE protein expression can be detected in HeLa cells and PC12 cells with expression level significantly increased under apoptosis condition; more importantly, abundant AChE protein expression was also detected in nucleus components (Figure 2B). The result was consistent with Confocal observation result, i.e., under apoptosis condition, AChE expression was increased and migrated to the nucleus. Wherein, α-Tubulin was used as the marker of cytoplasm components, and Histone H3 was used as the marker of nucleus components.

To observe the dynamic process in which AChE migrated to the nucleus during apoptosis, to provide some lights on the function of AChE, the inventors co-transfected two plasmids which expressed AChE-GFP fusion protein (pEGFP-*AChE*) and Histone H2b-RFP fusion protein (for nuclear localization) to HeLa cells, and then induced cell apoptosis with 100 µM H₂O₂; position changes of AChE after cell apoptosis initiation were traced and photographed by using living cell real-time imaging system. As expected, AChE-GFP was completely distributed in cytoplasm before adding H₂O₂, while Histone H2b-RFP was completely distributed in nucleus (Figure 2C, time 0); starting from 130 min after adding H₂O₂, especially after 150 min, AChE-GFP can be obviously seen both in cytoplasm and nucleus. It was worth noting that almost in the same time period, nucleus gradually became pyknotic, and presented significant apoptosis characteristics (Figure 2C). However, in untreated group co-transfected with pEGFP-AChE, pRFP-Histon H2b-RFP plasmids, AChE-GFP never migrated to the nucleus; moreover, in cells co-transfected with pEGFP-Tubulin, pRFP-Histone H2b plasmids, Tubulin-GFP never migrated to the nucleus after apoptosis induction (Figure 2C); it showed that AChE-GFP would migrated to the nucleus only when apoptosis was happening, and this nuclear translocation was not brought to the nucleus by GFP protein. It is suggested that this was active cell nuclear localization related with its function.

In conclusion, in normally growing cells, AChE, whether from background expression or exogenous overexpression, was distributed in cytoplasm; however, once cell started apoptosis program, AChE would migrate to the nucleus, and from the view of time, the nuclear transport time of AChE was close to the time when nucleus initiated pyknosis. Cell location of protein molecule was closely related with its function, thus, the discovery of new cell location of AChE provides an important clue for investigating its new functions.

### Example 3. The transient transfection of AChE located in nucleus led to cell death

Generally speaking, in cell apoptosis, proteins are translocated to cell nucleus, their functions can be divided into two aspects: first, regulating gene transcription (usually early entry); second, involving in karyopyknosis and chromosomal DNA breakage and degradation. To further explore biological significances of AChE in nuclear translocation during apoptosis, the inventors constructed AChE plasmid of human origin with nuclear localization signal (NLS), pEGFP -NLS- AChE. At 24h after the plasmid was transiently transfected to HeLa cell, observed under laser scanning confocal microscope. In cells transfected with pEGFP-AChE plasmid from control group, AChE-GFP fusion protein was distributed in cytoplasm (Figure 3A); above transfection positive cells underwent screening and sorting through flow cytometer, after which performed MTT growth curve determination for three days and found that there was no significant change in the growth status compared with control group expressing GFP (Figure 3B); meanwhile, the result of TUNEL assay was also negative (Figure 3C).

Above results indicated that in cells transfected with pEGFP-NLS-AChE plasmid, NLS-AChE-GFP fusion protein can be expressed in nucleus (Figure 3A); although not introducing external apoptosis stimuli, its cell metabolic rate was significantly reduced (Figure 3B).

The result of TUNEL assay shows that DNA of cells which expressed NLS-AChE-GFP in nucleus all occurred breakage, while cells negative for transfection were normal (Figure 3C), suggesting AChE expression in the nucleus can directly lead to cell death. Although the expression of Histone H2B-GFP fusion protein or GFP was also in the nucleus, its positive cells can grow normally (Figure 3B-C), indicating above observed cell death was not operated by transfection, nor non-specific death caused by protein transport to nucleus itself.

Above data shows that: after AChE occurred nuclear translocation during apoptosis process, it may perform important apoptosis-promoting function.

As to the reasons for AChE overexpression in nucleus would lead to chromosomal DNA breakage, the present inventor thought there were two parts: first, AChE protein had DNase activity, which directly cleaved chromosomal DNA after nuclear transport; second, nuclear transport of AChE activated certain important factor which was initially silenced due to spatial isolation, made its downstream DNase activated, thereby cleaving chromosomal DNA. To clarify above assumption, in an in vitro cell free system, purified AChE protein of human or mice origin (GenBank accession number of its sequence: NP_033729.1) (available from UC, SanDiego, US) and plasmid DNA (pEGFP-C1 plasmid) were coincubated under 37 °C, the results show that both two AChE proteins can effectively cleave plasmid DNA, cutting plasmid DNA from supercoil (SC) into Nick and Linear structures. There was a positive correlation between this cleaving ability and AChE protein concentration as well as time (Figure 3D-E).

Above results suggest that during apoptosis, the significance of increase in AChE expression and nuclear transport may lie in involving in chromosomal DNA degradation.

### Example 4. Identification on that AChE had DNase activity and exclusion of exogenous pollution

Since DNase I was served as a secretase, it was widely distributed in blood and digestive tract, involved in processes from DNA damage and repair to food digestion and clearance of exogenously infected virus, bacteria DNA and so on. Was above cleaving effect of AChE on DNA the illusion caused by exogenous DNA contamination? To clearly address at this problem, the present inventor used protein gel electrophoresis combining with silver staining method to analyze protein purity, showing that the main band of AChE protein was clear, with no evidence of other impurity bands (Figure 4A-B).

Was cleaving effect of AChE on DNA the non-specificity caused by system itself? To make clear this problem, first, BSA was used as negative control, found that BSA protein didn't have cleaving function of plasmid DNA (Figure 4C-D).

To further detect the specificity of AChE cleaving DNA, the inventors designed two experimental schemes as follows: scheme one, Protein G glass bead conjugated with AChE antibody was pre-incubated with AChE for 1h, and AChE in the system was depleted by centrifugation, and then incubated with plasmid DNA; scheme two, DNase I specific inhibitor G-Actin protein (Lacks SA. Deoxyribonuclease I in mammalian tissues. Specificity of inhibition by actin. J Biol Chem 1981; 256(6): 2644-8) was pre-incubated with AChE protein, and then whether AChE after incubation could cleave plasmid DNA was determined. The result of agarose gel electrophoresis shows that AChE specific antibody significantly weakened cleaving ability of AChE protein on plasmid DNA (Figure 4D); though G-Actin significantly inhibited DNase I activity, it almost had no effect on cleaving ability of AChE on DNA (Figure 4C).

In all of the above experiments, the inventors tried to exclude false positive results caused by exogenous DNase contamination, in order to minimize the possibility of exogenous DNase contamination, and strict control was introduced in each experiment. The reliability of hypothesis of the inventors, i.e., AChE had a new function of cleaving DNA, was verified through various ways from analyzing AChE protein purity to utilizing AChE antibody and DNase I inhibitor, etc.

Furthermore, the inventors artificially synthesized 32-72AA fragment (T41) and 32-138AA fragment (T107) of Homo AChE, and peptide fragments were co-incubated with mouse genome DNA for 6h according to different doses; and artificially synthesized 32-72AA fragment (T41) and 32-138AA fragment (T107) of Homo AChE, and the peptide fragments were coincubated with mouse genome DNA for different time. Results show that 32-72AA part (T41) of AChE was the same as control (water), which is not able to degrade and digest genome DNA; while 32-138AA fragment (T107) was able to degrade and digest genome DNA, and this degradation was dose-dependent and time-dependent (Figure 4E-F).

To further determine that AChE's ability as nuclease to cleave and degrade and digest DNA was independent of origin species of AChE, the inventors purchased commercial AChE isolated from Eel (available from SIGMA, Trade Name: Acetylcholinesterase from Electrophorus electricus (electric eel); Product Number : C2888-1KU), meanwhile purchased two bovine serum albumins (BSA) from different suppliers (BSA1 was available from Amresco, BSA2 was available from Sangon Biotech) as control, and found that the AChE from Eel can also degrade and digest DNA, while BSA as control does not have this nuclease activity, and this degradation was dose-dependent and time-dependent (Figure 4G-H).

### Example 5. Biochemical condition analysis in cleaving of DNA by AChE

It was reported that during apoptosis, intracellular metal cation concentration would be significantly increased (Rizzuto R, Pinton P, Ferrari D, Chami M, Szabadkai G, Magalhaes PJ et al. Calcium and apoptosis: facts and hypotheses. Oncogene 2003; 22(53): 8619-27; Zhivotovsky B, Cedervall B, Jiang S, Nicotera P, Orrenius S. Involvement of Ca2+ in the formation of high molecular weight DNA fragments in thymocyte apoptosis. Biochem Biophys Res Commun 1994; 202(1): 120-7). Studies show that activities of most enzymes are (including DNase) depended on metal cation.

To know more about the DNase property of AChE, the inventors detected the effect of metal cation on DNase activity of AChE. We found that when there was no Mg²⁺ and Ca²⁺ in a system , the activity of AChE cleaving DNA was very weak; when Mg²⁺ existed alone, its activity markedly restored; while Ca²⁺ was a weak activator, and when it existed alone, DNase activity of AChE was slightly increased (Figure 5A). This result was further confirmed by another experiment, i.e., when EDTA existed in a system, the activity of AChE cleaving DNA was significantly inhibited; different from EDTA, the inhibition effect of EGTA on this cleaving was not obvious (Figure 5A). Above data indicated that DNase activity of AChE is depended on Mg²⁺.

In addition to cation, pH environment in normally growing cell was also different from that in apoptotic cell (Lang F, Huber SM, Szabo I, Gulbins E. Plasma membrane ion channels in suicidal cell death. Arch Biochem Biophys 2007; 462(2): 189-94; Fernandez-Segura E, Canizares FJ, Cubero MA, Warley A, Campos A. Changes in elemental content during apoptotic cell death studied by electron probe X-ray microanalysis. Exp Cell Res 1999; 253(2): 454-62). For apoptosis related enzyme, its optimal pH value is between 7.5 and 9.5 (Deng G, Podack ER. Deoxyribonuclease induction in apoptotic cytotoxic T lymphocytes. FASEB J 1995; 9(8): 665-9).

To determine the optimal pH value for DNase activity of AChE, the inventors formulated a series of DNA Hydrolysis reaction buffer with different pH values (pH5-10). AChE was incubated with plasmid DNA (pEGFP-C1 plasmid) in above buffer, respectively, and found that in a relatively wide range of pH 7-10, AChE can effectively function to cleave DNA. While in the range of pH 5-6, its activity to cleave DNA reduced slightly, and the resulting amount of linear DNA was significantly decreased (Figure 5B).

AChE protein and DNase I presented similar binding curves. Software analysis show that Kd value between DNase I and DNA was about 1.14e-6M, Kd value between AChE and DNA was 1.53e-6 M. Kd value is inversely related to affinity, and results show that the affinity between AChE and DNA was about 74% of that of DNase I.

From above experiments, DNase activity of AChE is depended on Mg²⁺, while Ca²⁺ could only play a weak supporting role; in a relatively wide pH range of pH 7-10 (similar to in vivo pH environment during apoptosis), AChE can fully function to cleave DNA. And in the in vitro experiments, AChE showed relatively high affinity for DNA, and its affinity for DNA was about 74% of that of DNase I.

### Example 6. Searching and structural analyzing of sites where AChE shows DNase activity

Above results had already shown that AChE has DNase activity, and is capable of cleaving plasmids with supercoil conformation to present nick or linear status. However, classic function of AChE is to hydrolyze acetylcholine, having acetylcholinesterase activity. Both acetylcholinesterase and DNase belong to esterase, and are capable of hydrolyzing ester bond and making it breaking. Then, did active centre of acetylcholinesterase of AChE play a role in its DNase? The inventors pre-incubated AChE protein and AChE esterase inhibitors Huperzine A, Tacrine and Donepezil (Huperzine A, Tacrine and Donepezil were purchased from Sigma), respectively, after which plasmid DNA was added. Results show that the three inhibitors all can significantly inhibit esterase activity of AChE (Figure 6A), but didn't significantly affect its DNase activity (Figure 6B). It suggests that active site of AChE esterase may not function in its DNase. To further make clear this inference, catalytic triplet amino acids of AChE esterase active site were subject to site directed mutagenesis (S234A, E365A and H478A), and constructed to a GFP vector with NLS sequence, to obtain pEGFP-NLS-AChE S234A/E365A/H478A mutant plasmid. At 24h after this plasmid was transiently transfected to HeLa cell, esterase activity assay confirmed that esterase activity of mutated AChE protein was already lost. If acetylcholinesterase active site of AChE played a role in its DNA-cleaving function, the mutated protein would lose DNA-cleaving function, and even overexpressing and nuclear localization without other apoptosis stimuli induction would also lose apoptosis-triggering capability. However, the overexpressing of mutated protein cannot remedy apoptosis (Figure 13). It can be seen that acetylcholinesterase active site of AChE is irrelevant to its DNase function.

### Example 7. AChE triggers various tumor cell apoptosis after entering nucleus

The inventors constructed pEGFP-*NLS-AChE* plasmid, which expresses fusion protein NLS-AChE; AChE protein which can enter the nucleus under the mediation of NLS.

Test results after pEGFP-*NLS-AChE* plasmid was transfected to colon cancer SW620 cell can be seen in Figure 7; from electron microscope pictures (Figure 7A), it can be seen that compared with empty plasmid control (pEGFP), *NLS-AChE* transfection positive cells present extremely striking apoptosis. The result of MTT assay shows that OD₅₇₀ value of cell was close to 0.

Test results after pEGFP-*NLS-AChE* plasmid was transfected to cervical cancer HeLa cell can be seen Figure 8. From electron microscope pictures (Figure 8A), it can be seen that compared with empty plasmid control (pEGFP), *NLS-AChE* transfection positive cells present extremely striking apoptosis. In Figure 8B, positive for Hochest staining, positive for GFP staining and being able to overlap, suggesting that expressed fusion protein entered cell. It can be seen from Figure 8C that compared with empty plasmid control (pEGFP), *NLS-AChE* transfection positive cells present extremely striking apoptosis, and the result of MTT assay shows that OD₅₇₀ value of cell was close to 0. Alone *AChE* (no *NLS*) transfection positive cells also present certain apoptosis, but the apoptosis efficiency was less than that of *NLS-AChE* transfection positive cells.

Test results after pEGFP-*NLS-AChE* plasmid was transfected to liver cancer BEL7404 cell can be seen in Figure 9. From electron microscope pictures (Figure 9A), it can be seen that compared with empty plasmid control (pEGFP), *NLS-AChE* transfection positive cells present extremely striking apoptosis. In Figure 9B, positive for Hochest staining, positive for GFP staining and being able to overlap, suggesting that expressed fusion protein entered cell. It can be seen from Figure 9C that compared with empty plasmid control (pEGFP), *NLS-AChE* transfection positive cells present extremely striking apoptosis, and the result of MTT assay shows that OD₅₇₀ value of cell was close to 0. Alone *AChE* (no *NLS*) transfection positive cells also present certain apoptosis, but apoptosis efficiency was less than that of *NLS-AChE* transfection positive cells.

As can be seen, various tumor cell apoptosis can be effectively triggered after *NLS-AChE* transfection, and MTT assay shows that apoptosis rate was about 100%. Therefore, the present invention was fully demonstrated by various cell lines, confirming that protein AChE expressed by a novel apoptosis-inducing gene *AChE* triggering various tumor cell apoptosis after entering nucleus.

### Example 8. AChE protein level reduced in tumor patients

Given that genome DNA degradation was a prominent feature of cell apoptosis, and AChE protein also had DNA degradation function, it can be seen that whichever tumor cell was *NLS-AChE* gene transfected to, NLS-AChE protein would all transport to the nucleus after expression and directly triggered cell death.

In view of the above findings, it is suggested that *AChE* gene was a cancer suppressor gene, whose expression in cancer tissues may be inhibited. Therefore, the inventors determined *AChE* gene expression in cancer tissues in 24 liver cancer patients by immunohistochemical method. Results are as Figure 10, it is found that in cancer tissues in 24 liver cancer patients, AChE protein level showing serious decline accounted for about 75% (compared with para-carcinoma tissues).

Above data suggest that if *AChE* genes are forcely expressed in tumor cells, and its protein are localized to the nucleus, the tumor cells would be dead and lose the ability of tumor formation.

Above all, immunohistochemical results of AChE protein levels in liver cancer and para-carcinoma tissues support the conclusion that *AChE* gene is a cancer suppressor gene.

### Example 9. NLS-AChE gene inhibited tumor formation ability of tumor cells

To further identify the effect of *NLS-AChE* in inhibiting cancer. The inventors performed the in vivo mice experiments. After *NLS-AChE* gene was transfected to liver cancer BEL7404 cell, NLS-AChE protein was expressed and transported into the nucleus. This recombinantly expressed cell was subcutaneously implanted near forelimbs of Bal B/C nude mice, and tumor formation of mice was observed.

Results are shown in Figure 11, and Figure 11A shows the subcutaneous presence of BEL7404 cells transfected with pEGFP or pEGFP-*NLS*-*AChE* plasmid in nude mice at 24hr, 72hr after flow cytometry sorting observed by stereo fluorescence microscope. At 72hr, compared with pEGFP-transfected group, fluorescence signals of cells in pEGFP-*NLS-AChE* group are very weak, suggesting a lot of cell loss.

Figure 11B is the curve of tumor volume change in 28 days, in which cells were implanted subcutaneously in nude mice after flow cytometry screening. Cells in pEGFP-*NLS-AChE* group never formed tumor; tumor volume tended to linearly increase from day 7 to day 25 after cells in pEGFP group formed tumor. Calculation formula of tumor volume: V=(length×width²)π/4.

Figures 11C-E are photographs of nude mice showing tumor formation on day 28 after flow cytometry screening. Mice of Row 1, implanted with pEGFP-transfected cells, significantly formed tumor (the third, fourth from the right, tumor was to large to be torn ), and tumors isolated from 4 mice can be seen in Figure 11D, respectively; cell characteristics of tumor tissues can be seen in Figure 11E; Mice of Row 2, implanted pEGFP-*NLS-A ChES*-transfected cells, formed no tumor.

Therefore, AChE protein promoted BEL7404 cell death, and the ability of BEL7404 to form tumor subcutaneously in mude mice was completely inhibited.

### Example 10. Identification of the fragment in AChE protein molecule having apoptosis-promoting function

To find key fragment in AChE protein molecule playing apoptosis-promoting function, the inventors constructed various deletion mutants of AChE protein molecule (Figure 12A); and 32-138AA fragment of human AChE protein (T107).

Mutant-containing plasmids (containing coding sequences for EGFP-NLS-AchE mutants) were transfected to HeLa cells, respectively; transfection positive cells were sorted by flow cytometer and implanted in 96-well plate, and the fluorescent inverted phase contrast microscope at 72h showed cell morphological changes (x200). Results can be seen from Figure 12B, and 2-138AA fragment or longer fragment both made cell present death features.

MTT assay was performed on above-mentioned cells and cell growth curve was plotted. Results can be seen from Figure 12C, and 2-138AA fragment had apoptosis-promoting function of full-length AChE, while 2-72AA fragment does not have this function.

The expression of 32-138AA fragment of human AChE (T107) plus nuclear localization signal NLS in HeLa cells also had significant killing effect on in vitro cultured tumor cells, making cells present death features.

2-138AA, 32-138AA fragments can fully perform apoptosis-promoting function of AChE after transport to the nucleus; moreover, 2-138AA, 32-138AA fragments were common segments of AChER, AChES, AchEH. For this, the three types of AChE all had the function of promoting the apoptosis of tumor cells.

### Example 11. Screening method

### (1) Method 1

As Example 4, human AChE protein and plasmid DNA (pEGFP-C1 plasmid) were co-incubated in 37°C, resulting in a coincubation system, and the following two groups were set:
Test group: above-mentioned co-incubation system treated by candidate materials;
Control group: above-mentioned coincubation system not treated by candidate materials.

The efficiency of AChE cleaving plasmid DNA at appropriate time after treatment was observed, if compared with control group, the efficiency of test group is improved by more than 20%, suggesting that the candidate materials are potential materials useful for promoting cell apoptosis. If compared with control group, the efficiency of test group is reduced by more than 20%, suggesting that the candidate materials are potential materials useful for inhibiting cell apoptosis.

### (2) Method 2

HeLa cells endogenously expressing AChE protein were selected, and treated by hydrogen peroxide (50 µM) to cause apoptosis, and the following two groups were set:
Test group: above-mentioned cells treated by candidate materials;
Control group: above-mentioned cells not treated by candidate materials.

The expression quantity of AChE protein was determined, if compared with control group, the expression quantity of AChE protein of the text group is significantly decreased by more than 30%, suggesting that the candidate materials are potential materials useful for inhibiting cell apoptosis.

Above-mentioned siRNA1 and siRNA2 were used as candidate materials, and after treating above cells with the candidate materials, the expression quantity of AChE protein was tested, and the results show that the expression quantity of AChE protein was significantly inhibited by more than 30%. Therefore, the inhibitor can be served as a useful material for inhibiting cell apoptosis.

### (3) Method 3

Screening method is as follows:
HeLa cells were plated in 96-well plate with a 60% density, and after complete adherence, the cells were fixed with 4% PFA for 10 min, and permeabilized with 0.1% sodium citrate +0.1%Triton X-100 for 10 min, for twice. After PBS washing, cells were divided into five groups, as follows:
   HeLa cell group: normal cell control; fixed and treated for permeabilization as above;
   AChE protein group: HeLa cell, treated with AChE protein (1µg/µl) after fixation and permeabilization treatment;
   BSA control group: HeLa cell, containing AChE protein, treated with BSA protein (2.5µg/µl) after fixation and permeabilization treatment;
   AChE protein + candidate materials group: HeLa cell, containing AChE protein, treated with candidate materials after fixation and permeabilization treatment;
   Standard positive reference group: HeLa cell, containing AChE protein, treated with DNase after fixation and permeabilization treatment; the reference group was set for reflecting general conditions of DNA breakage.

Cells of each group were incubated for 1-2 hr in 37°C; Washed twice with PBS, and fixed with 4%PFA for 10 min. Tunnel reagent was added, incubated for 1 hr in 37°C; and then washed three times with PBS, stained with Hoechest. Observation was performed by fluorescence microscope.

AChE protein group was compared with BSA group, and it can be seen that intracellular DNA present obvious breakage in AChE protein group, as Figure 14.

If observing after treatment with said candidate materials, compared with AChE protein group, DNA breakage in AChE protein + candidate materials group reduced or there was no breakage, it is suggested that the candidate materials were AChE protein inhibitors, and are capable of inhibiting nuclease activity of AChE protein. AChE protein inhibitors screened from the method can be potentially used as drugs for preventing or relieving Alzheimer's disease (AD) or Parkinson's disease (PD).

### Discussion

AChE has attracted wide attention and in-depth study for a long time, as an important molecule to hydrolyze neurotransmitter acetylcholine in cholinergic nerve synapse and neuromuscular junction. However, in addition to nervous system, AChE is also widely distributed in a variety of other cell types, such as epithelial cell, blood cell and endothelial cell, indicating that AChE is also involved in some "nonclassical functions". Reported "nonclassical functions" of AChE include neuritogenesis, cell proliferation (Xiang AC, Xie J, Zhang XJ. Acetylcholinesterase in intestinal cell differentiation involves G2/M cell cycle arrest. Cell Mol Life Sci 2008; 65(11): 1768-79), cell adhesion, synaptogenesis, amyloid fibril aggregation, hematopoiesis and thrombopoiesis. Specific mechanisms of these functions are not clear yet.

In recent years, although reports have shown that when inducing cell apoptosis, the phenomenon of increase in acetylcholinesterase (AChE) expression can be observed; but how to explain this phenomenon is not known; it may be explained as product of apoptosis process, or as by-product caused by cell metabolism disorder, or as involving in apoptosis process. Therefore, what role does AChE play in apoptosis process is still unknown yet.

The inventors unexpectedly found that besides increase in expression quantity, AChE also had migration tendency to the nucleus during apoptosis process. In normally growing cells, AChE, whether from background expression or exogenous overexpression, was distributed in cytoplasm; however, once cell started apoptosis program, AChE would migrate to the nucleus, and from the view of time, the nuclear transport time of AChE was close to the time when nucleus initiated pyknosis. Cell location of protein molecule was closely related with its function, and thus, the discovery of new cell location of AChE provided an important clue for investigating its new functions.

The inventors further investigated the time and opportunity of AChE transport to nucleus, and thought that AChE may be involved in karyopyknosis and chromosomal DNA breakage and degradation. To further verify this assumption, the inventors inserted a nuclear localization signal peptide (NLS) at N-terminal of AChE, so that the expressed fusion protein had the property of active nuclear translocation. Through observation, the inventors found that AChE expression in the nucleus can directly initiate apoptosis without introducing external apoptosis stimuli, and TUNEL staining for chromosomal DNA was positive. After utilizing MTT analyzing on NLS-AChE-transfected and FACS-positively enriched cells, the inventors found that compared with cells expressing non-nuclear localized AChE or GFP, survival rate of NLS-AChE-transfected cells was significantly reduced. Although the expression of Histone H2B-GFP fusion protein or GFP was also in the nucleus, its positive cells can grow normally, indicating that the above observed cell death was not a non-specific death resulted from the operation by transfection, or from protein transporting to nucleus itself.

As to the reasons for AChE overexpression in nucleus would lead to chromosomal DNA breakage, the inventors thought there were two parts: first, AChE protein had DNase activity, which directly cleaved chromosomal DNA after entering the nucleus; second, AChE's entering the nucleus activated certain important factor which was silenced due to spatial isolation, made its downstream DNase activated, thereby cleaving chromosomal DNA. To clarify above assumption, in an in vitro cell free system, the purified AChE protein of human or mice origin and plasmid DNA were co-incubated in 37 °C, and the results show that both two AChE proteins can effectively cleave plasmid DNA, cutting plasmid DNA from supercoil (SC) into Nick and Linear structures. There is a positive correlation between this cleaving ability and AChE protein concentration as well as time.

Is the cleaving effect of AChE on DNA the non-specificity caused by system itself? To make clear this problem, first BSA was used as a negative control, and it is found that BSA protein didn't have cleaving function of plasmid DNA (Figures 3d and e). To further detect the specificity of AChE cleaving DNA, the inventors designed two experimental schemes as follows: scheme one, Protein G glass bead conjugated with AChE antibody was pre-incubated with AChE for 1h, and AChE in the system was depleted by centrifugation, before incubation with plasmid DNA; scheme two, DNase I specific inhibitor G-Actin protein 19 was pre-incubated with AChE protein, and then determined whether AChE after incubation could cleave plasmid DNA. The result of agarose gel electrophoresis shows that AChE specific antibody significantly weakened the cleaving ability of AChE protein on plasmid DNA (Figure 4d); though G-Actin significantly inhibited DNase I activity, it almost had no effect on cleaving ability of AChE on DNA (Figure 4c).

Meanwhile, the inventors detected environment characteristics in which AChE cleaving DNA. DNase activity of AChE was Mg2+-dependent, while Ca2+ could only play a weak supporting role (Figure 5a). In a relatively wide pH range of pH 7.0-10.0 (similar to in vivo pH environment during apoptosis), AChE can fully play its function of cleaving DNA (Figure 5b). Moreover, according to the result of Bio Core T100 assay which determined dissociation constant between AChE and DNA, AChE showed relatively high affinity for DNA, and its affinity for DNA was about 74% of that of DNase I.

Is there a relationship between DNase activity of AChE and its esterase activity to hydrolyze acetylcholine (ACh)? By the experiement wherein three AChE esterase inhibitors (Huperzine A, Tacrine and Donepezil) were added to an in vitro cleaving system and the AChE esterase activity site mutations were overexpressed intracellular, the inventors found all the three inhibitors had no significant influence on its DNA-cleaving activity(Figure 6b). On the other hand, the experiment in which amino acids of AChE esterase active sites were subject to site directed mutagenesis (S234A, E365A and H478A, respectively) and connected to expression vector with NLS sequence to express in HEK 293T, also confirmed this result, i.e., active center at which AChE functioned DNase activity was different from the center at which it functioned esterase activity to hydrolyze ACh.

In conclusion, AChE shows increased expression and enters the nucleus during cell apoptosis. Expressing AChE in the nucleus using NLS signal peptide will directly lead to cell apoptosis. In vitro experiments show that AChE has the ability to bind and cleave DNA. siRNA for AChE can significantly reduce the degree of chromosomal DNA breakage during apoptosis, suggesting that AChE is involved in chromosomal DNA degradation and clearance during apoptosis and plays an important role in it.

In the present invention, all the literature mentioned are incorporated by reference in the present application, as if each reference was individually incorporated by reference. It should also be understood that after reading the teachings of the present invention, the skilled in the art can make various modifications or changes to the present invention, and these equivalents also fall within the present application as defined by the appended claims scope.

## Claims

1. Use of acetylcholinesterase or the agonist or antagonist thereof for preparing a composition for regulating the stability of nucleic acid or regulating cell apoptosis.

2. The use of claim 1, wherein the acetylcholinesterase or the agonist thereof is used for preparing a composition which damages nucleic acid and/or promotes cell apoptosis.

3. The use of claim 2, wherein the agonist of the acetylcholinesterase is selected from the group consisting of: agents which direct acetylcholinesterase or its encoding gene to enter a nucleus, magnesium ions, calcium ions or materials which can provide magnesium ions and/or calcium ions.

4. The use of claim 2, wherein the nucleic acid is a tumor- or virus-related nucleic acid, or the cell is a tumor cell.

5. The use of claim 2, wherein the acetylcholinesterase or the agonist thereof is used for preparing a composition which inhibits a tumor.

6. The use of claim 1, wherein the inhibitor of the acetylcholinesterase is used for preparing a composition which protects nucleic acid and/or inhibits cell apoptosis.

7. The use of claim 6, wherein the inhibitor is selected from the group consisting of: nucleic acid inhibitors, protein inhibitors, antibodies, ligands, proteolytic enzymes, protein-binding molecules, EDTA.

8. The use of claim 7, wherein the nucleic acid inhibitor is selected from the group consisting of: the interfering molecule of SEQ ID NO: 1 or SEQ ID NO: 2.

9. An isolated acetylcholinesterase fragment or variant, which is selected from:
(b) a polypeptide of aa 32-138 of SEQ ID NO:5;
(c) a polypeptide of aa 2-138 of SEQ ID NO:5;
(d) a polypeptide of aa 2-191 of SEQ ID NO:5;
(e) a polypeptide of aa 2-247 of SEQ ID NO:5;
(f) a polypeptide of ss 2-398 of SEQ ID NO:5;
(g) a polypeptide aa 32-578 of SEQ ID NO:5;
(h) a polypeptide of aa 32-579 of the amino acid sequence shown in GenBank accession number NP_033729.1;
(i) a polypeptide having an amino acid sequence of the polypeptide of any of (e) - (g), wherein variations occur in position 234, 365 and/or 478;
(j) a polypeptide formed with substitution, deletion or addition of one or more amino acid residues in the amino acid sequence of the polypeptide of any of (b)-(i), and having the function of the polypeptide of SEQ ID NO: 5;
(k) a fragment of the polypeptide of any of (b)- (j) having the function of the polypeptide of SEQ ID NO: 5; or
(l) a polypeptide having the function of the polypeptide of SEQ ID NO: 5 and containing the amino acid sequence of the polypeptide of any of (b)-(k).

10. An isolated polynucleotide, containing a nucleotide sequence selected from the group consisting of:
(1) a polynucleotide encoding the polypeptide of claim 9;
(2) a polynucleotide complementary to the polynucleotide (1).

11. The polynucleotide of claim 10, wherein the nucleotide sequence of the polynucleotide is selected from:
(i) a polynucleotide of the nucleotide sequence at position 4-414 of SEQ ID NO:4;
(ii) a polynucleotide of the nucleotide sequence at position 4-573 of SEQ ID NO:4;
(iii) a polynucleotide of the nucleotide sequence at position 4-741 of SEQ ID NO:4;
(iv) a polynucleotide of the nucleotide sequence at position 4-1194 of SEQ ID NO:4; or
(v) a polynucleotide of the nucleotide sequence at position 94-1734 of SEQ ID NO:4.

12. A vector containing the polynucleotide of claim 10 or 11.

13. A genetic engineered host cell, containing the vector of claim 12; or being integrated with the polynucleotide of claim 11 or 12 in its genome.

14. A method for preparing acetylcholinesterase fragment or variant of claim 9, comprising:
(a) culturing the host cell of claim 13 under conditions suitable for expression;
(b) isolating the acetylcholinesterase fragment or variant of claim 9 from the culture.

15. Use of the acetylcholinesterase fragment or variant of claim 9, the polynucleotide of claim 10 or 11, the vector of claim 12, or the host cell of claim 13, for preparing a composition for regulating the stability of nucleic acid or regulating cell apoptosis, or for preparing an anti-tumor drug.

16. An acetylcholinesterase inhibitor for protecting nucleic acid and/or inhibiting cell apoptosis, having the nucleotide sequence of SEQ ID NO: 1 or SEQ ID NO: 2.

17. A material which regulates nucleic acid stability or regulates cell apoptosis, comprising:
(1) an acetylcholinesterase or the agonist or antagonist thereof; and
(2) a nuclear importer, which directs the acetylcholinesterase or the agonist or antagonist thereof to enter the nucleus.

18. The material of claim 17, wherein, further comprises:
(3) a cell- or tissue-targeting material; and/or
(4) a protein transduction domain polypeptide.

19. The material of claim 17 or 18, wherein, (1),(2),(3) and/or (4) are combined by means of covalent linkage, coupling, the action of molecular force, charge adsorption, adherence, encapsulation or embedding.

20. The material of claim 17 or 18, wherein, the nuclear importer is selected from the group consists of: a nuclear localization signal or karyopherin or the core fragments thereof, steroid hormone, virus, viral protein or virus-like particle, cationic polymer, radionuclide, nanosphere.

21. The material of claim 17 or 18, wherein the material is a fusion protein comprising an acetylcholinesterase and a nuclear importer.

22. The material of claim 21, wherein, the fusion protein contains:
an acetylcholinesterase and a nuclear localization signal.

23. A polynucleotide encoding the material of any of claims 21-22.

24. An expression construct containing the polynucleotide of claim 23.

25. The expression construct of claim 24, wherein further comprises a specific promoter operably linked to the polynucleotide of the fusion protein, and the specific promoter is selected from the group consist of: cell-specific promoter, tissue-specific promoter, or nucleus-specific promoter.

26. Use of the material of any of claims 17-22, the polynucleotide of claim 23 or the expression construct of claim 24 or 25, for damaging nucleic acid and/or promoting cell apoptosis; or for preparing a composition which damages nucleic acid and/or promotes cell apoptosis.

27. The use of claim 26, wherein the nucleic acid is a tumor- or virus-related nucleic acid, or the cell is a tumor cell.

28. The use of claim 27, wherein the material is used for preparing an anti-tumor drug.

29. A composition for damaging nucleic acid and/or promoting cell apoptosis, comprising:
the acetylcholinesterase fragment or variant of claim 9, the vector of claim 12, the material of any of claims 17-22, or the polynucleotide according to claim 23, or the expression construct according to claim 24 or 25, and
a pharmaceutically or physiologically acceptable carrier.

30. A composition for damaging nucleic acid and/or promoting cell apoptosis, comprising:
an acetylcholinesterase, the acetylcholinesterase fragment or variant of claim 9, or the vector of claim 12, or the material of any of claims 17-22, or the polynucleotide according to claim 23, or the expression construct according to claim 24 or 25, and
magnesium ions and/or calcium ions.

31. The composition of claim 29 or 30, wherein the pH value of the composition is 5-10.

32. A method for damaging nucleic acid, comprising: contacting an acetylcholinesterase or the agonist thereof or a vector expressing acetylcholinesterase or the agonist thereof, or the acetylcholinesterase fragment or variant of claim 9,or the vector of claim 12, with the nucleic acd.

33. A method for promoting cell apoptosis, comprising:
treating cells with an acetylcholinesterase or the agonist thereof, or a vector expressing acetylcholinesterase, or the material of any of claims 17-22, or the polynucleotide of claim 23, or the expression construct of claim 24 or 25, or the acetylcholinesterase fragment or variant of claim 9, or the vector of claim 12; or
expressing acetylcholinesterase or the agonist thereof, or the material of any of claims 16-21; or the acetylcholinesterase fragment or variant of claim 9 by the cells; or
directing acetylcholinesterase or the acetylcholinesterase fragment or variant of claim 9 into the nucleus.

34. A method for protecting nucleic acid or inhibiting cell apoptosis, comprising:
treating cells with the inhibitor of acetylcholinesterase;
expressing the inhibitor of acetylcholinesterase by the cells; or
preventing the acetylcholinesterase from entering the nucleus.

35. A method for screening a potential drug which regulates cell apoptosis or nucleic acid stability, wherein the method comprises:
(1) treating a system expressing or containing acetylcholinesterase or the fragment or variant thereof with a candidate material; and
(2) detecting the transcription, expression or activity of the acetylcholinesterase or the fragment or variant thereof in the system;
wherein, if the candidate material can improve the transcription, expression or activity of the acetylcholinesterase or the fragment or variant thereof, it is indicated that the candidate material is a potential material to damage nucleic acid or promote cell apoptosis; if the candidate material can reduce the transcription, expression or activity of the acetylcholinesterase or the fragment or variant thereof, it is indicated that the candidate material is a potential material to protect nucleic acid or inhibit cell apoptosis.

36. The method of claim 35, wherein the system expressing or containing an acetylcholinesterase or the fragment or variant thereof is a cell, and step (2) also includes:
observing nuclear transport of the acetylcholinesterase or the fragment or variant thereof or its encoding gene; if the proportion of the acetylcholinesterase or the fragment or variant thereof or its encoding gene in cell nucleus increases, it is suggested that the candidate material is a potential material to damage nucleic acid or promote cell apoptosis; if the proportion of the acetylcholinesterase or the fragment or variant thereof or its encoding gene in cell nucleus decreases, it is suggested that the candidate material is a potential material to protect nucleic acid or inhibit cell apoptosis; or,
step (2) also includes:
observing DNA breakage in cell nucleus; if DNA breakage increases, it is suggested that the candidate material is a potential material to damage nucleic acid or promote cell apoptosis; if DNA breakage decreases, it is suggested that the candidate material is a potential material to protect nucleic acid or inhibit cell apoptosis.

37. A method for screening a potential drug which inhibits cell apoptosis or regulates nucleic acid stability, comprising:
(1) treating a cell expressing or containing an acetylcholinesterase or the fragment or variant thereof in a test group with a candidate material; and
(2) detecting intracellular DNA breakage in the test group, and comparing the result with that of a control group, wherein the control group is the cells expressing or containing an acetylcholinesterase or the fragment or variant thereof without adding the candidate material;
wherein, if DNA breakage in the test group significantly increases compared with control group, it is suggested that the candidate material is a potential drug to inhibit cell apoptosis or nucleic acid stability.

38. The method of claim 37, wherein the cell is a fixed cell; and/or the cell is a cell with membrane treated for permeabilization.

39. The method of any of claims 35-38, wherein the acetylcholinesterase or the fragment or variant thereof is an acetylcholinesterase fragment including no esterase active site.

40. A method for screening a potential drug which regulates cell apoptosis or nucleic acid stability, comprising:
(1') treating a system with a candidate material, wherein the system contains an acetylcholinesterase or the fragment or variant thereof as well as nucleic acid; and
(2') detecting nucleic acid breakage within the system; if the nucleic acid breakage increases, it is suggested that the candidate material is a potential material to damage nucleic acid or promote cell apoptosis; if the nucleic acid breakage decreases, it is suggested that the candidate material is a potential material to protect nucleic acid or inhibit cell apoptosis.


SEQUENCE LISTING

<110>  Shanghai Institutes For Biological Sciences, Chinese Academy Of
Sciences

<120>  The use of ACHE as nuclease

<130>  113693 PCWO

<160>  6

<170>  PatentIn version 3.3

<210>  1
<211>  25
<212>  RNA
<213>  artificial sequence

<220>
<221>  misc_feature
<223>  oligonucleotide
<400>  1
aagucucccg cguugaugag ggccu                                          25

<210>  2
<211>  25
<212>  RNA
<213>  artificial sequence

<220>
<221>  misc_feature
<223>  oligonucleotide
<400>  2
aagaagcggc caucguacac gucca                                          25

<210>  3
<211>  51
<212>  DNA
<213>  Simian Virus
<400>  3
atggctccaa agaagaagcg taaggtagga ccaaagaaga agcgtaaggt t             51

<210>  4
<211>  1845
<212>  DNA
<213>  Homo Sapiens
<400>  4
atgaggcccc cgcagtgtct gctgcacacg ccttccctgg cttccccact ccttctcctc      60
ctcctctggc tcctgggtgg aggagtgggg gctgagggcc gggaggatgc agagctgctg     120
gtgacggtgc gtgggggccg gctgcggggc attcgcctga agacccccgg gggccctgtc     180
tctgctttcc tgggcatccc ctttgcggag ccacccatgg gaccccgtcg ctttctgcca     240
ccggagccca agcagccttg gtcaggggtg gtagacgcta caaccttcca gagtgtctgc     300
taccaatatg tggacaccct atacccaggt tttgagggca ccgagatgtg gaaccccaac      360
cgtgagctga gcgaggactg cctgtacctc aacgtgtgga caccataccc ccggcctaca     420
tcccccaccc ctgtcctcgt ctggatctat gggggtggct tctacagtgg ggcctcctcc     480
ttggacgtgt acgatggccg cttcttggta caggccgaga ggactgtgct ggtgtccatg     540
aactaccggg tgggagcctt tggcttcctg gccctgccgg ggagccgaga ggccccgggc     600
aatgtgggtc tcctggatca gaggctggcc ctgcagtggg tgcaggagaa cgtggcagcc     660
ttcgggggtg acccgacatc agtgacgctg tttggggaga gcgcgggagc cgcctcggtg     720
ggcatgcacc tgctgtcccc gcccagccgg ggcctgttcc acagggccgt gctgcagagc     780
ggtgccccca atggaccctg ggccacggtg ggcatgggag aggcccgtcg cagggccacg     840
cagctggccc accttgtggg ctgtcctcca ggcggcactg gtgggaatga cacagagctg     900
gtagcctgcc ttcggacacg accagcgcag gtcctggtga accacgaatg gcacgtgctg     960

cctcaagaaa gcgtcttccg gttctccttc gtgcctgtgg tagatggaga cttcctcagt 1020
gacaccccag aggccctcat caacgcggga gacttccacg gcctgcaggt gctggtgggt 1080
gtggtgaagg atgagggctc gtattttctg gtttacgggg ccccaggctt cagcaaagac 1140
aacgagtctc tcatcagccg ggccgagttc ctggccgggg tgcgggtcgg ggttccccag 1200
gtaagtgacc tggcagccga ggctgtggtc ctgcattaca cagactggct gcatcccgag 1260
gacccggcac gcctgaggga ggccctgagc gatgtggtgg gcgaccacaa tgtcgtgtgc 1320
cccgtggccc agctggctgg gcgactggct gcccagggtg cccgggtcta cgcctacgtc 1380
tttgaacacc gtgcttccac gctctcctgg cccctgtgga tgggggtgcc ccacggctac 1440
gagatcgagt tcatctttgg gatccccctg gacccctctc gaaactacac ggcagaggag 1500
aaaatcttcg cccagcgact gatgcgatac tgggccaact ttgcccgcac aggggatccc 1560
aatgagcccc gagaccccaa ggccccacaa tggcccccgt acacggcggg ggctcagcag 1620
tacgttagtc tggacctgcg gccgctggag gtgcggcggg ggctgcgcgc ccaggcctgc 1680
gccttctgga accgcttcct ccccaaattg ctcagcgcca ccgacacgct cgacgaggcg 1740
gagcgccagt ggaaggccga gttccaccgc tggagctcct acatggtgca ctggaagaac 1800
cagttcgacc actacagcaa gcaggatcgc tgctcagacc tgtga 1845

<210> 5
<211> 614
<212> PRT
<213> Homo Sapiens

<400> 5
Met Arg Pro Pro Gln Cys Leu Leu His Thr Pro Ser Leu Ala Ser Pro
1               5                   10                  15
Leu Leu Leu Leu Leu Leu Trp Leu Leu Gly Gly Gly Val Gly Ala Glu
            20                  25                  30
Gly Arg Glu Asp Ala Glu Leu Leu Val Thr Val Arg Gly Gly Arg Leu
        35                  40                  45
Arg Gly Ile Arg Leu Lys Thr Pro Gly Gly Pro Val Ser Ala Phe Leu
    50                  55                  60
Gly Ile Pro Phe Ala Glu Pro Pro Met Gly Pro Arg Arg Phe Leu Pro
65                  70                  75                  80
Pro Glu Pro Lys Gln Pro Trp Ser Gly Val Val Asp Ala Thr Thr Phe
                85                  90                  95
Gln Ser Val Cys Tyr Gln Tyr Val Asp Thr Leu Tyr Pro Gly Phe Glu
            100                 105                 110
Gly Thr Glu Met Trp Asn Pro Asn Arg Glu Leu Ser Glu Asp Cys Leu
        115                 120                 125
Tyr Leu Asn Val Trp Thr Pro Tyr Pro Arg Pro Thr Ser Pro Thr Pro
    130                 135                 140
Val Leu Val Trp Ile Tyr Gly Gly Gly Phe Tyr Ser Gly Ala Ser Ser
145                 150                 155                 160
Leu Asp Val Tyr Asp Gly Arg Phe Leu Val Gln Ala Glu Arg Thr Val
                165                 170                 175
Leu Val Ser Met Asn Tyr Arg Val Gly Ala Phe Gly Phe Leu Ala Leu
            180                 185                 190
Pro Gly Ser Arg Glu Ala Pro Gly Asn Val Gly Leu Leu Asp Gln Arg
        195                 200                 205
Leu Ala Leu Gln Trp Val Gln Glu Asn Val Ala Ala Phe Gly Gly Asp
    210                 215                 220
Pro Thr Ser Val Thr Leu Phe Gly Glu Ser Ala Gly Ala Ala Ser Val
225                 230                 235                 240
Gly Met His Leu Leu Ser Pro Pro Ser Arg Gly Leu Phe His Arg Ala
                245                 250                 255
Val Leu Gln Ser Gly Ala Pro Asn Gly Pro Trp Ala Thr Val Gly Met
            260                 265                 270
Gly Glu Ala Arg Arg Arg Ala Thr Gln Leu Ala His Leu Val Gly Cys
        275                 280                 285
Pro Pro Gly Gly Thr Gly Gly Asn Asp Thr Glu Leu Val Ala Cys Leu
    290                 295                 300
Arg Thr Arg Pro Ala Gln Val Leu Val Asn His Glu Trp His Val Leu
305                 310                 315                 320
Pro Gln Glu Ser Val Phe Arg Phe Ser Phe Val Pro Val Val Asp Gly

325                 330                 335
Asp Phe Leu Ser Asp Thr Pro Glu Ala Leu Ile Asn Ala Gly Asp Phe
            340                 345                 350
His Gly Leu Gln Val Leu Val Gly Val Val Lys Asp Glu Gly Ser Tyr
        355                 360                 365
Phe Leu Val Tyr Gly Ala Pro Gly Phe Ser Lys Asp Asn Glu Ser Leu
    370                 375                 380
Ile Ser Arg Ala Glu Phe Leu Ala Gly Val Arg Val Gly Val Pro Gln
385                 390                 395                 400
Val Ser Asp Leu Ala Ala Glu Ala Val Val Leu His Tyr Thr Asp Trp
                405                 410                 415
Leu His Pro Glu Asp Pro Ala Arg Leu Arg Glu Ala Leu Ser Asp Val
            420                 425                 430
Val Gly Asp His Asn Val Val Cys Pro Val Ala Gln Leu Ala Gly Arg
        435                 440                 445
Leu Ala Ala Gln Gly Ala Arg Val Tyr Ala Tyr Val Phe Glu His Arg
    450                 455                 460
Ala Ser Thr Leu Ser Trp Pro Leu Trp Met Gly Val Pro His Gly Tyr
465                 470                 475                 480
Glu Ile Glu Phe Ile Phe Gly Ile Pro Leu Asp Pro Ser Arg Asn Tyr
                485                 490                 495
Thr Ala Glu Glu Lys Ile Phe Ala Gln Arg Leu Met Arg Tyr Trp Ala
            500                 505                 510
Asn Phe Ala Arg Thr Gly Asp Pro Asn Glu Pro Arg Asp Pro Lys Ala
        515                 520                 525
Pro Gln Trp Pro Pro Tyr Thr Ala Gly Ala Gln Gln Tyr Val Ser Leu
    530                 535                 540
Asp Leu Arg Pro Leu Glu Val Arg Arg Gly Leu Arg Ala Gln Ala Cys
545                 550                 555                 560
Ala Phe Trp Asn Arg Phe Leu Pro Lys Leu Leu Ser Ala Thr Asp Thr
                565                 570                 575
Leu Asp Glu Ala Glu Arg Gln Trp Lys Ala Glu Phe His Arg Trp Ser
            580                 585                 590
Ser Tyr Met Val His Trp Lys Asn Gln Phe Asp His Tyr Ser Lys Gln
        595                 600                 605
Asp Arg Cys Ser Asp Leu
    610

<210>  6
<211>  633
<212>  PRT
<213>  artificial sequence
<220>
<221>  MISC_FEATURE
<223>  fusion protein

<400>  6
Met Ala Pro Lys Lys Lys Arg Lys Val Gly Pro Lys Lys Lys Arg Lys
1               5                   10                  15
Val Lys Leu Met Arg Pro Pro Gln Cys Leu Leu His Thr Pro Ser Leu
            20                  25                  30
Ala Ser Pro Leu Leu Leu Leu Leu Leu Trp Leu Leu Gly Gly Gly Val
        35                  40                  45
Gly Ala Glu Gly Arg Glu Asp Ala Glu Leu Leu Val Thr Val Arg Gly
    50                  55                  60
Gly Arg Leu Arg Gly Ile Arg Leu Lys Thr Pro Gly Gly Pro Val Ser
65                  70                  75                  80
Ala Phe Leu Gly Ile Pro Phe Ala Glu Pro Pro Met Gly Pro Arg Arg
                85                  90                  95
Phe Leu Pro Pro Glu Pro Lys Gln Pro Trp Ser Gly Val Val Asp Ala
            100                 105                 110
Thr Thr Phe Gln Ser Val Cys Tyr Gln Tyr Val Asp Thr Leu Tyr Pro
        115                 120                 125

Gly Phe Glu Gly Thr Glu Met Trp Asn Pro Asn Arg Glu Leu Ser Glu
    130                 135                 140
Asp Cys Leu Tyr Leu Asn Val Trp Thr Pro Tyr Pro Arg Pro Thr Ser
145                 150                 155                 160
Pro Thr Pro Val Leu Val Trp Ile Tyr Gly Gly Gly Phe Tyr Ser Gly
                165                 170                 175
Ala Ser Ser Leu Asp Val Tyr Asp Gly Arg Phe Leu Val Gln Ala Glu
            180                 185                 190
Arg Thr Val Leu Val Ser Met Asn Tyr Arg Val Gly Ala Phe Gly Phe
        195                 200                 205
Leu Ala Leu Pro Gly Ser Arg Glu Ala Pro Gly Asn Val Gly Leu Leu
    210                 215                 220
Asp Gln Arg Leu Ala Leu Gln Trp Val Gln Glu Asn Val Ala Ala Phe
225                 230                 235                 240
Gly Gly Asp Pro Thr Ser Val Thr Leu Phe Gly Glu Ser Ala Gly Ala
                245                 250                 255
Ala Ser Val Gly Met His Leu Leu Ser Pro Pro Ser Arg Gly Leu Phe
            260                 265                 270
His Arg Ala Val Leu Gln Ser Gly Ala Pro Asn Gly Pro Trp Ala Thr
        275                 280                 285
Val Gly Met Gly Glu Ala Arg Arg Arg Ala Thr Gln Leu Ala His Leu
    290                 295                 300
Val Gly Cys Pro Pro Gly Gly Thr Gly Gly Asn Asp Thr Glu Leu Val
305                 310                 315                 320
Ala Cys Leu Arg Thr Arg Pro Ala Gln Val Leu Val Asn His Glu Trp
                325                 330                 335
His Val Leu Pro Gln Glu Ser Val Phe Arg Phe Ser Phe Val Pro Val
            340                 345                 350
Val Asp Gly Asp Phe Leu Ser Asp Thr Pro Glu Ala Leu Ile Asn Ala
        355                 360                 365
Gly Asp Phe His Gly Leu Gln Val Leu Val Gly Val Val Lys Asp Glu
    370                 375                 380
Gly Ser Tyr Phe Leu Val Tyr Gly Ala Pro Gly Phe Ser Lys Asp Asn
385                 390                 395                 400
Glu Ser Leu Ile Ser Arg Ala Glu Phe Leu Ala Gly Val Arg Val Gly
                405                 410                 415
Val Pro Gln Val Ser Asp Leu Ala Ala Glu Ala Val Val Leu His Tyr
            420                 425                 430
Thr Asp Trp Leu His Pro Glu Asp Pro Ala Arg Leu Arg Glu Ala Leu
        435                 440                 445
Ser Asp Val Val Gly Asp His Asn Val Val Cys Pro Val Ala Gln Leu
    450                 455                 460
Ala Gly Arg Leu Ala Ala Gln Gly Ala Arg Val Tyr Ala Tyr Val Phe
465                 470                 475                 480
Glu His Arg Ala Ser Thr Leu Ser Trp Pro Leu Trp Met Gly Val Pro
                485                 490                 495
His Gly Tyr Glu Ile Glu Phe Ile Phe Gly Ile Pro Leu Asp Pro Ser
            500                 505                 510
Arg Asn Tyr Thr Ala Glu Glu Lys Ile Phe Ala Gln Arg Leu Met Arg
        515                 520                 525
Tyr Trp Ala Asn Phe Ala Arg Thr Gly Asp Pro Asn Glu Pro Arg Asp
    530                 535                 540
Pro Lys Ala Pro Gln Trp Pro Pro Tyr Thr Ala Gly Ala Gln Gln Tyr
545                 550                 555                 560
Val Ser Leu Asp Leu Arg Pro Leu Glu Val Arg Arg Gly Leu Arg Ala
                565                 570                 575
Gln Ala Cys Ala Phe Trp Asn Arg Phe Leu Pro Lys Leu Leu Ser Ala
            580                 585                 590
Thr Asp Thr Leu Asp Glu Ala Glu Arg Gln Trp Lys Ala Glu Phe His
        595                 600                 605
Arg Trp Ser Ser Tyr Met Val His Trp Lys Asn Gln Phe Asp His Tyr
    610                 615                 620
Ser Lys Gln Asp Arg Cys Ser Asp Leu

625                                630